(19) Europäisches Patentamt
European Patent Office
Office européen des brevets

(11) **EP 1 490 016 B1**

(12) # EUROPÄISCHE PATENTSCHRIFT

(45) Veröffentlichungstag und Bekanntmachung des Hinweises auf die Patenterteilung:
**06.06.2007 Patentblatt 2007/23**

(21) Anmeldenummer: **03714814.5**

(22) Anmeldetag: **13.03.2003**

(51) Int Cl.:
***A61K 8/91*** *(2006.01)* ***A61Q 5/00*** *(2006.01)*
***A61Q 5/06*** *(2006.01)* ***A61Q 5/12*** *(2006.01)*

(86) Internationale Anmeldenummer:
**PCT/EP2003/002577**

(87) Internationale Veröffentlichungsnummer:
**WO 2003/080001 (02.10.2003 Gazette 2003/40)**

(54) **KATIONISCHE POLYMERISATE UND DEREN VERWENDUNG IN KOSMETISCHEN FORMULIERUNGEN**

CATIONIC POLYMERS AND THE USE THEREOF IN COSMETIC FORMULATIONS

POLYMERISATS CATIONIQUES ET LEUR UTILISATION DANS DES FORMULATIONS COSMETIQUES

(84) Benannte Vertragsstaaten:
**AT BE BG CH CY CZ DE DK EE ES FI FR GB GR HU IE IT LI LU MC NL PT RO SE SI SK TR**

(30) Priorität: **21.03.2002 DE 10212705**
**19.06.2002 DE 10227500**

(43) Veröffentlichungstag der Anmeldung:
**29.12.2004 Patentblatt 2004/53**

(73) Patentinhaber: **BASF Aktiengesellschaft**
**67056 Ludwigshafen (DE)**

(72) Erfinder:
- **WOOD, Claudia**
  **69469 Weinheim (DE)**
- **ANGEL, Maximilian**
  **67105 Schifferstadt (DE)**
- **CHRISSTOFFELS, Lysander**
  **67117 Limburgerhof (DE)**

(56) Entgegenhaltungen:
**EP-A- 1 123 942** **WO-A-00/49998**
**DE-A- 19 907 587** **US-A- 4 048 301**
**US-A- 4 240 450** **US-A- 4 380 600**

- **PATENT ABSTRACTS OF JAPAN vol. 2000, no. 16, 8. Mai 2001 (2001-05-08) & JP 2001 019830 A (MITSUBISHI PLASTICS IND LTD), 23. Januar 2001 (2001-01-23)**
- **PATENT ABSTRACTS OF JAPAN vol. 012, no. 297 (C-519), 12. August 1988 (1988-08-12) & JP 63 063739 A (MITSUBISHI RAYON CO LTD), 22. März 1988 (1988-03-22)**
- **PATENT ABSTRACTS OF JAPAN vol. 2000, no. 24, 11. Mai 2001 (2001-05-11) & JP 2001 181354 A (LION CORP), 3. Juli 2001 (2001-07-03)**
- **PATENT ABSTRACTS OF JAPAN vol. 018, no. 522 (P-1808), 30. September 1994 (1994-09-30) & JP 06 180859 A (MITSUBISHI RAYON CO LTD), 28. Juni 1994 (1994-06-28)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 490 016 B1

Printed by Jouve, 75001 PARIS (FR)

**Beschreibung**

Die vorliegende Erfindung betrifft kationische Polymerisate, Verfahren zu deren Herstellung, sowie deren Verwendung in kosmetischen Formulierungen. Die kationischen Pfropfpolymerisate sind erhältlich, durch Polymerisation von quaternisiertkationischen, radikalisch polymerisierbaren Monomeren und optional weiteren radikalisch copolymerisierbaren Monomeren in Gegenwart einer polyetherhaltigen Verbindung mit der Maßgabe, dass die Umsetzung in Gegenwart geringer Wassermengen von unter 20 Gew.-% am Gesamtreaktionsgemisch erfolgt.

DE 197 14 714 A1 beschreibt lineare Blockpolymere aus Polyethylenglykol und kationischen Monomeren, die unter Einsatz von Makroinitiatoren synthetisiert werden. Die Verbindungen werden als polymere Tenside verwendet. Pfropfreaktionen, die zu verzweigten Polymeren führen sind nicht beschrieben und bei dem beschriebenen Verfahren - nach Aussage der Erfinder - zu vernachlässigen (Lieske A und Jaeger W (1998) Macromol Chem Phys 199:255-260; siehe insbesondere S. 256; linke Spalte, letzter Absatz: "Side reactions, particularly grafting onto the PEG chain and transfer reactions leading to DADMAC homopolymer, are neglectable").

DE-A 29 24 663 und US 4,380,600 beschreiben die radikalische Polymerisation ethylenisch ungesättigter Monomere in wässriger Lösung unter Verwendung von wasserlöslichen Polymeren als Dispergierungsmittel. Als wasserlösliches Polymer wird u.a. Polyethylenglykol verwendet. Als ethylenisch ungesättigtes Monomer werden auch quaternäre Ammoniumverbindungen eingesetzt. Bei dem beschriebenen Verfahren handelt es sich um eine Wasser-in-Wasser ("W/W") - Emulsionspolymerisation, die erhebliche Wasseranteile aufweist. So werden die ethylenisch ungesättigten Monomere in einem Gewichtsverhältnis von 3 bis 150 Teile zu 100 Teilen Wasser eingesetzt. Das Gewichtsverhältnis von ethylenisch ungesättigten Monomer zu dem wasserlöslichen Polymer (z.B. Polyethylenglykol) beträgt 1: 5 bis 5:1. Der hohe Wasseranteil bedingt, dass bei dieser Verfahrensführung kein oder nur ein sehr geringer Anteil an Pfropfung der ethylenisch ungesättigten Monomere auf das wasserlösliche Polymer erfolgt. Das wasserlösliche Polymer (z.B. Polyethylenglykol) hat hier die Funktion eines Dispergierungsmittels für die wässrige Dispersion.

EP-A 0 183 466 beschreibt Verfahren zur Polymerisation wasserlöslicher Monomere in wässriger Lösung in Gegenwart eines Dispergierungsmittels und eines Salzes. Als Dispergierungsmittel kann u.a. Polyethylenglykol verwendet werden. Wasserlösliche Monomere schließen bestimmte kationische Monomere ein. Der für eine "W/W"-Emulsionspolymerisation typische hohe Wasseranteil bedingt, dass bei dieser Verfahrensführung kein oder nur ein sehr geringer Anteil an Pfropfung der ethylenisch ungesättigten Monomere auf das wasserlösliche Polymer erfolgt. Das wasserlösliche Polymer (z.B. Polyethylenglykol) hat hier die Funktion eines Dispergierungsmittels. Eine Pfropfung der Monomere auf das Dispergierungsmittel ist nicht erwähnt. Die erhaltenen Verbindungen werden als Flockungsmittel zur Abwasserbehandlung oder in der Papierindustrie eingesetzt. Verwendung in kosmetischen Zubereitungen ist nicht beschrieben.

EP-A1 0 880 548 und DE 195 21 096 A1 beschreiben Verfahren zur Herstellung von Dispersionen wasserlöslicher Vinylpolymere und Stabilisatoren zur Durchführung des Verfahrens. Als Stabilisatoren werden Pfropfpolymere bestehend aus einem Rückgrat aus Polyethylenoxid und Pfropfästen aus kationischen, bevorzugt quarternären Vinylpolymeren beschrieben. Die Synthese des Stabilisators wie in Beispiel 1 beschrieben erfolgt in wässriger Lösung. Die Gewichtsverhältnis ethylenisch ungesättigtes Monomer:Polyethylenglykol:Wasser betragen 24:30:270. Eine Verwendung dieser Stabilisatoren in kosmetischen Zubereitungen ist nicht erwähnt. Nachteilig bei dem beschriebenen Verfahren ist der hohe Wasseranteil im Reaktionsgemisch. Bei dieser Verfahrensführung erfolgt kein oder nur ein sehr geringer Anteil an Pfropfung der ethylenisch ungesättigten Monomere auf das wasserlösliche Polymer d.h. es liegen Polymere aus dem kationischen Monomer und Polyethylenglykol zu einem erheblichen Anteil individuell ohne Pfropfung nebeneinander vor (vgl. Aussage in Lieske A & Jaeger W (1998) Macromol Chem Phys 199:255-260; s.o.). Dies beeinträchtigt die Qualität des Produktes, insbesondere seine Eignung in der Kosmetik. Polyethylenglykol hat hier die Funktion eines Dispergierungsmittels.

EP-A 1 123 942 beschreibt die Polymerisation anionischer (carboxylischer) ethylenisch ungesättigter Monomere in einer Schmelze aus Polyethylenglykol. Die im Polymer enthaltenen Carboxyl-Gruppen werden anschließend durch Zugabe eines Alkylenimins zumindest teilweise umgesetzt. Bevorzugt werden dabei primäre Aminogruppen erhalten. Auch sekundäre sind unter Umständen möglich. Tertiäre oder quarternäre sind nicht beschrieben und nach dem beanspruchten Verfahren auch nicht zugänglich.

DD 117 326 beschreibt die Herstellung quaternisierter Pfropfpolymere mittels einem zweistufigen Verfahren. Es erfolgt zunächst eine Polymerisation nicht-ionischer ethylenisch ungesättigter Monomere (3-Chlor-2-hydroxypropylacrylat bzw. Vinylimidazol) in einer Schmelze aus Polyethylenglykol. Die Quaternisierung erfolgt ausgehend von dem erhaltenen Polymer in einem zweiten Schritt durch Umsatz mit Triethylamin bzw. Methyliodid. Die Polymere finden ihren Einsatz in der Herstellung von fotografischen Gelatine-Halogensilberemulsionsschichten. Eine Verwendung im Bereich der Kosmetik ist nicht beschrieben. Das beschriebene Verfahren hat Nachteile aus wirtschaftlicher und verfahrenstechnischer Sicht. Zum einen ist die Prozessführung aufgrund des zweistufigen Verfahrens aufwendig. Zum anderen kann das Verfahren - beispielsweise infolge eines nicht vollständigen zweiten Umsetzungsschrittes - zu Polymeren mit nicht reproduzierbaren Eigenschaften führen. Der zweite Umsetzungsschritt erfordert zudem zum Teil giftige Verbindungen, die die Herstellung weiterhin erschweren und - insbesondere bei einer Verwendung des Polymers im z.B. kosmetischen

Anwendungsbereich - eine Reinigung erzwingen.

WO 00/49998 beschreibt Polymerisationsverfahren von Vinylestern in polyetherhaltigen Verbindungen wie beispielsweise Polyethylenglykol. Zusätzlich können - optional - weitere Monomere wie kationische Monomere eingesetzt werden. Das Verfahren beinhaltet die anschließende Verseifung des Polymerisates. Bei den beschriebenen Polymeren ist der Anteil der optional zusätzlich eingesetzten Monomere auf maximal 50 % beschränkt (s. S. 9 / Z. 32 ff.). Bevorzugt beträgt er 0 bis 20 %.

US 3,990,459 beschreibt kosmetische Zusammensetzungen, die gepfropfte kationische Polymere beinhalten. Besagte gepfropfte kationische Polymere werden durch Copolymerisation von 3 bis 95 Gew.-% Dimethylaminoethylmethacrylat, 2 bis 50 Gew.-% Polyethylenglykol enthalten und 3 bis 95 Gew.-% eines weiteren Monomeres wie z.B. N-Vinylpyrrolidon erhalten. Eine Quaternisierung von Dimethylaminoethylmethacrylat kann beispielsweise mit Dimethylsulfat vorgenommen werden. Bei den hier beschriebenen Polymeren ist der Polyethylenglykolanteil entsprechend dem Anspruch nicht größer als 50 Gew.-%. In den erfindungsgemäßen Beispielen liegt er sogar deutlich darunter und zwar bei 10 Gew.-%.

US 4,408,301 beschreibt Schampoo-Zusammensetzungen, die gepfropfte kationische Polymere beinhalten. Besagte gepfropfte kationische Polymere werden durch Copolymerisation von 3 bis 95 Gew.-% N-Vinylpyrrolidon, 3 bis 95 Gew.-% Dimethylaminoethylmethacrylat und 2 bis 50 Gew.-% Polyethylenglykol erhalten. Eine Quaternisierung von Dimethylaminoethylmethacrylat kann beispielsweise mit Dimethylsulfat vorgenommen werden. Bei den hier beschriebenen Polymeren ist der Polyethylenglykolanteil entsprechend dem Anspruch nicht größer als 50 Gew.-%. In den erfindungsgemäßen Beispielen liegt er sogar deutlich darunter und zwar bei 8,15 bzw. 8,81 Gew.-%.

DE 2 623 692 beschreibt Haarfärbemittel, die gepfropfte kationische Polymere beinhalten. Besagte gepfropfte kationische Polymere werden durch Copolymerisation von N-Vinylpyrrolidon, Dimethylaminoethylmethacrylat und Polyethylenglykol erhalten. Eine Quaternisierung von Dimethylaminoethylmethacrylat kann beispielsweise mit Dimethylsulfat vorgenommen werden. Bevorzugt werden die Pfropfpolymere durch Copolymerisation von 3 bis 95 Gew.-% N-Vinylpyrrolidon, 3 bis 95 Gew.-% Dimethylaminoethylmethacrylat und 2 bis 50 Gew.-% Polyethylenglykol erhalten. Bei den hier beschriebenen Polymeren ist der Polyethylenglykolanteil entsprechend dem Anspruch nicht größer als 50 Gew.-%. In den erfindungsgemäßen Beispielen liegt er sogar deutlich darunter und zwar bei 8,15 bzw. 8,81 Gew.-%.

Für die Konditionierung und Festigung von keratinösen Substanzen wie Haar, Nägel und Haut werden seit Jahren auch synthetische Polymere eingesetzt. Anforderungen an Haarkonditioniermittel sind z.B. eine starke Reduktion der erforderlichen Kämmkraft im nassen wie auch im trockenen Haar, gute Entwirrung beim ersten Durchkämmen (engl. "Detangling") und gute Verträglichkeit mit weiteren Formulierungskomponenten. Anforderungen an Haarfestigerharze sind z.B. eine starke Festigung bei hoher Luftfeuchtigkeit, Elastizität, Auswaschbarkeit vom Haar und Verträglichkeit mit weiteren Formulierungskomponenten. Schwierigkeiten bereitet die Kombination verschiedener Eigenschaften. So zeigen Polymere mit guten Festigungseigenschaften oftmals geringe Elastizitäten, so dass bei mechanischer Beanspruchung der Frisur die Festigungswirkung durch Schädigung des Polymerfilm oft erheblich beeinträchtigt wird. Zudem werden synthetische Polymere in kosmetischen Formulierungen, die Pigmente oder kosmetisch wirksame Aktivkomponenten enthalten, als Verträglichkeitsvermittler zur Erreichung einer homogenen, stabilen Formulierung eingesetzt.

Trotz der umfangreichen Bemühungen besteht nach wie vor Verbesserungsbedarf bei Polymeren zur Erzeugung elastischer Frisuren bei gleichzeitig starker Festigung auch bei hoher Luftfeuchtigkeit, guter Auswaschbarkeit und gutem Griff des Haares. Der Verbesserungsbedarf besteht ebenso bei Polymeren zur Erzeugung von gut kämmbarem, entwirrbarem Haar und zur Konditionierung von Haut und Haar in ihren sensorisch erfassbaren Eigenschaften wie Griff, Volumen, Handhabbarkeit usw. Ferner sind klare wässrige Zubereitungen dieser Polymere wünschenswert, die sich demnach durch eine gute Verträglichkeit mit anderen Formulierungesbestandteilen auszeichnen.

Es bestand demnach die Aufgabe, neue Polymere für insbesondere haarkosmetische Formulierungen bereitzustellen, die einerseits der Frisur eine starke Festigung bei gleichzeitig hoher Elastizität und andererseits dem Haar eine gute Kämmbarkeit bei gleichzeitig voluminösem Aussehen verleihen und mit Wasser klare Zubereitungen ergeben.

Die Aufgabe wird durch die Bereitstellung der erfindungsgemäßen Polymerisate gelöst.

Ein erster Gegenstand der Erfindung betrifft die Verwendung von kationischen Polymerisaten, erhältlich durch Polymerisation von

3 bis 30 Gew.-% mindestens eines quaternären Stickstoff enthaltenden radikalisch polymerisierbaren Monomers (a1) und/oder eines direkten Vorproduktes (a2) desselben,

in Gegenwart von 70 bis 97 Gew.-% mindestens einer polyetherhaltigen Verbindung (b) wie in Anspruch 1 definiert und

gegebenenfalls 0 bis 15 Gew.-% eines oder mehrerer weiterer radikalisch polymerisierbarer Monomere (c) mit einer Wasserlöslichkeit oberhalb 60 g/l bei 25°C und

gegebenenfalls 0 bis 15 Gew.-% eines oder mehrerer weiterer radikalisch polymerisierbarer Monomeren (d) mit einer Wasserlöslichkeit unterhalb 60 g/l bei 25°C

wobei der Wassergehalt im Reaktionsgemisch während der Polymerisation weniger als 20 Gew.-% beträgt, und wobei bei Verwendung eines Vorproduktes (a2) dieses im Anschluss an oder während der Polymerisation zumindest teilweise in eine Verbindung mit quaternärem Stickstoff (a2') umgesetzt wird, wobei das molare Verhältnis der Summe der Monomeren (a1), (a2') und (c) zu der Summe der Monomeren (d) mindestens 2 zu 1 beträgt, und wobei sich die Gew.-% der einzelnen Komponenten a1 und/oder a2, b und gegebenenfalls c und d auf jeweils 100 Gew.-% addieren,

in kosmetischen Zubereitungen.

Ein weiterer Gegenstand der Erfindung betrifft Polymerisate, erhältlich durch Polymerisation von

3 bis 30 Gew.-% mindestens eines kationischen, quarternären radikalisch polymerisierbaren Monomers (a1) und

in Gegenwart von 70 bis 97 Gew.-% mindestens einer polyetherhaltigen Verbindung (b) wie in Anspruch 1 definiert und

gegebenenfalls 0 bis 15 Gew.-% eines oder mehrerer weiterer radikalisch polymerisierbarer Monomere (c) mit einer Wasserlöslichkeit oberhalb 60 g/l bei 25°C und

gegebenenfalls 0 bis 15 Gew.-% eines oder mehrerer weiterer radikalisch polymerisierbarer Monomere (d) mit einer Wasserlöslichkeit unterhalb 60 g/l bei 25°C,

wobei das molare Verhältnis der Summe der Monomeren (a1) und (c) zu der Summe der Monomeren (d) mindestens 2 zu 1 beträgt, wobei der Wassergehalt im Reaktionsgemisch während der Polymerisation weniger als 20 Gew.-% beträgt, und wobei sich die Gew.-% der einzelnen Komponenten a1, b und gegebenenfalls c und d auf jeweils 100 Gew.-% addieren.

Umfasst ist auch die Verwendung besagter erfindungsgemäßer Polymerisate in unten aufgeführten Anwendungen, insbesondere in kosmetischen Zubereitungen.

Ein weiterer Gegenstand der Erfindung betrifft, Verfahren zur Herstellung der erfindungsgemäßen Polymerisaten, dadurch gekennzeichnet, dass

3 bis 30 Gew.-% mindestens eines kationischen, quarternären radikalisch polymerisierbaren Monomers (a1)

in Gegenwart von 70 bis 97 Gew.-% mindestens einer polyetherhaltigen Verbindung (b) wie in Anspruch 1 definiert und

gegebenenfalls 0 bis 15 Gew.-% eines oder mehrerer weiterer radikalisch polymerisierbarer Monomere (c) mit einer Wasserlöslichkeit oberhalb 60 g/l bei 25°C und

gegebenenfalls 0 bis 15 Gew.-% eines oder mehrerer weiterer radikalisch polymerisierbarer Monomere (d) mit einer Wasserlöslichkeit unterhalb 60 g/l bei 25°C,

polymerisiert wird, wobei das molare Verhältnis der Summe der Monomeren (a1) und (c) zu der Summe der Monomeren (d) mindestens 2 zu 1 beträgt, wobei der Wassergehalt im Reaktionsgemisch während der Polymerisation weniger als 20 Gew.-% beträgt, und wobei sich die Gew.-% der einzelnen Komponenten a1, b und gegebenenfalls c und d auf jeweils 100 Gew.-% addieren.

Die erfindungsgemäßen Polymerisate weisen eine hervorragende Trocken- und Nasskämmbarkeit der mit ihnen behandelten Haare auf. Weitere Vorteile sind unter anderem der weiche Griff und die antistatischen Eigenschaften der damit behandelten Oberflächen wie Textilien, Haar, Haut, Papier, Faser- und Vliesmaterialien, aber auch anderen Oberflächen. Pigmenthaltige oder Zubereitungen mit kosmetisch wirksamen Aktivkomponenten werden durch die Polymerisate stabilisiert. Ein weiterer Vorteil ist, dass mit den erfindungsgemäßen Polymerisaten wässrige Zusammensetzungen wie Haarshampoos und Waschgele klar formuliert werden können. Darüberhinaus können die Polymerisate verwendet werden in Form von wässrigen oder wässrig/alkoholischen Lösungen, als wässrige Emulsion, Mikroemulsion,

Dispersion, opaque oder transparente Gele oder Aerosole.

Von wesentlicher Bedeutung für die hervorragenden konditionierenden Eigenschaften ist ein Anteil von mindestens 70 Gew.-% an polyetherhaltigen Verbindungen an den erfindungsgemäßen Polymerisaten, die sich dadurch von den in US 3,990,459, US 4,048,301 und DE 26 23 692 offenbarten Polymerisaten deutlich abheben. Die dort beschriebenen Polyere sind den erfindungsgemäßen z.B. hinsichtlich der Kemmkraftabnahme bei haarkosmetischen Anwendungen deutlichen unterlegen (siehe Vergleichsbeispiel 3).

Bei der Herstellung der erfindungsgemäß verwendeten Polymerisate kann es während der Polymerisation zu einer Pfropfung auf die polyetherhaltigen Verbindungen (b) kommen, was zu den vorteilhaften Eigenschaften der Polymerisate führen kann. Je nach Pfropfungsgrad sind unter den erfindungsgemäß verwendeten Polymerisaten sowohl reine Pfropfpolymerisate als auch Mischungen der o.g. Pfröpfpolymerisate mit ungepfropften polyetherhaltigen Verbindungen und Homo- oder Copolymerisaten der Monomeren (a1) und gegebenenfalls (ä2), (c) und (d) zu verstehen. Dabei heben sich die erfindungsgemäßen Polymerisate bezüglich ihrer Eigenschaften deutlich von Mischungen ab, bei denen die Polymerisation in Gegenwart größerer Wassermengen realisiert wird (siehe Vergleichsversuch 1) oder bei denen die Polyetherkomponente erst nach der Polymerisation der Monomere zugegeben wird (siehe Vergleichsversuch 2). Es sind jedoch auch andere Mechanismen als Pfropfung vorstellbar, die diese veränderten, vorteilhaften Eigenschaften bedingen können.

Als Polyether (b) werden Polymerisate der allgemeinen Formel I verwendet,

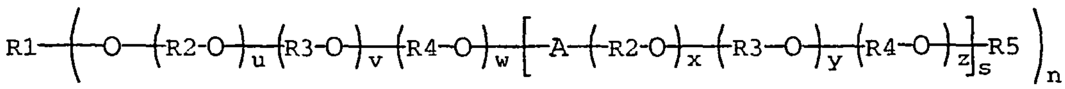

(I)

in der die Variablen unabhängig voneinander folgende Bedeutung haben:

$R^1$ Wasserstoff, $C_1$-$C_{24}$-Alkyl, $R^6$-C(=O)-, $R^6$-NH-C(=O)-, Polyalkoholrest;

$R^5$ Wasserstoff, $C_1$-$C_{24}$-Alkyl, $R^6$-C(=O)-, $R^6$-NH-C(=O)- ;

$R^2$ bis $R^4$ unabhängig voneinander für $-(CH_2)_2-$, $-(CH_2)_3-$, $-(CH_2)_4-$, $-CH_2-CH(R^6)-$, $-CH_2-CHOR^7-CH_2-$;

$R^6$ $C_1$-$C_{24}$-Alkyl;

$R^7$ Wasserstoff, $C_1$-$C_{24}$-Alkyl, $R^6$-C(=O)-, $R^6$-NH-C(=O)-;

A -C(=O)-O, -C(=O)-B-C(=O)-O, $-CH_2-CH(-OH)-B-CH(-OH)-CH_2-O$, -C(=O)-NH-B-NH-C(=O)-O,

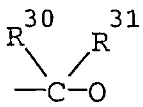

;

B $-(CH_2)_t-$, Arylen, ggf. substituiert;

$R^{30}$, $R^{31}$ unabhängig voneinander für Wasserstoff, $C_1$-$C_{24}$-Alkyl, $C_1$-$C_{24}$-Hydroxyalkyl, Benzyl oder Phenyl;

n 1 wenn $R^1$ kein Polyalkoholrest ist oder

n 1 bis 1000 wenn $R^1$ ein Polyakoholrest ist

s 0 bis 1000;

t 1 bis 12;

u 1 bis 5000;

v 0 bis 5000;

w 0 bis 5000:

x 0 bis 5000;

y 0 bis 5000;

z 0 bis 5000.

Die endständigen primären oder sekundären Hydroxylgruppen der auf Basis von Polyalkylenoxiden hergestellten Polyether sowie die sekundären OH-Gruppen von Polyglycerin können dabei sowohl in ungeschützter Form frei vorliegen als auch mit Alkoholen, wie beispielsweise Mono-, Di-, Tri- oder Polyalkoholen, einer Kettenlänge $C_1$-$C_{24}$ bzw. mit Carbonsäuren einer Kettenlänge $C_1$-$C_{24}$ verethert bzw. verestert und gegebenenfalls vernetzt werden oder mit Isocyanaten, Diisocyanaten oder Triisocyanaten zu Urethanen umgesetzt und gegebenenfalls vernetzt werden.

Als Alkylreste für $R^1$ und $R^5$ bis $R^7$ seien verzweigte oder unverzweigte $C_1$-$C_{24}$-Alkylketten, bevorzugt Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, 2-Ethylhexyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosyl genannt.

Als bevorzugte Vertreter der oben genannten Alkylreste seien verzweigte oder unverzweigte $C_1$ bis $C_{12}$-, besonders bevorzugt $C_1$ bis $C_6$-Alkylketten genannt.

Das Molekulargewicht der Polyether liegt im Bereich kleiner 1000000 (nach Zahlenmittel), bevorzugt im Bereich von 300 bis 100000, besonders bevorzugt im Bereich von 500 bis 50000, ganz besonders bevorzugt im Bereich von 600 bis 20000.

Homopolymere aus Ethylenoxid oder Copolymere aus Ethylenoxid und Propylenoxid sind besonders bevorzugt, wobei das molare Verhältnis von Ethylenoxid zu Propylenoxid bevorzugt in einem Bereich von 1:9 bis 9:1 liegt. Vorteilhafterweise verwendet man Homopolymerisate des Ethylenoxids oder Copolymerisate, mit einem Ethylenoxidanteil von 9 bis 99 mol-%. Für die bevorzugt einzusetzenden Ethylenoxidpolymerisate beträgt somit der Anteil an einpolymerisiertem Ethylenoxid 10 bis 100 mol-%. Als Comonomer für diese Copolymerisate kommen Propylenoxid, Butylenoxid und/oder Isobutylenoxid in Betracht. Geeignet sind beispielsweise Copolymerisate aus Ethylenoxid und Propylenoxid, Copolymerisate aus Ethylenoxid und Butylenoxid sowie Copolymerisate aus Ethylenoxid, Propylenoxid und mindestens einem Butylenoxid. Der Ethylenoxidanteil der Copolymerisate beträgt vorzugsweise 10 bis 99 mol-%, der Propylenoxidanteil 1 bis 90 mol-% und der Anteil an Butylenoxid in den Copolymerisaten 1 bis 30 mol-%. Neben geradkettigen können auch verzweigte Homo- oder Copolymerisate als polyetherhaltige Verbindungen (b) verwendet werden.

Verzweigte Polymerisate können hergestellt werden, indem man beispielsweise an Polyalkoholresten, z.B. an Pentaerythrit, Glycerin oder an Zuckeralkoholen wie D-Sorbit und D-Mannit aber auch an Polysaccharide wie Cellulose und Stärke, Ethylenoxid und gegebenenfalls noch Propylenoxid und/oder Butylenoxide anlagert. Die Alkylenoxid-Einheiten können im Polymerisat statistisch verteilt sein oder in Form von Blöcken vorliegen.

Es ist aber auch möglich, Polyester von Polyalkylenoxiden und aliphatischen oder aromatischen Dicarbonsäuren, z.B. Oxalsäure, Bernsteinsäure, Adipinsäure und Terephthalsäure mit Molmassen von 1500 bis 25000, wie z.B. beschrieben in EP-A-0 743 962, als polyetherhaltige Verbindung zu verwenden. Des weiteren können auch Polycarbonate durch-Umsetzung von Polyalkylenoxiden mit Phosgen oder Carbonaten wie z.B. Diphenylcarbonat, sowie Polyurethane durch Umsetzung von Polyalkylenoxiden mit aliphatischen und aromatischen Diisocyanaten verwendet werden.

Besonders bevorzugt werden als Polyether (b) Polymerisate der allgemeinen Formel I mit einem mittleren Molekulargewicht von 300 bis 100.000 (nach dem Zahlenmittel), in der die Variablen unabhängig voneinander folgende Bedeutung haben:

$R^1$ Wasserstoff, $C_1$-$C1_2$-Alkyl, $R^6$-C(=O)-, $R^6$-NH-C(=O)-, Polyalkoholrest;

$R^5$ Wasserstoff, $C_1$-$C_{12}$-Alkyl, $R^6$-C(=O)-, $R^6$-NH-C(=O)-;

$R^2$ bis $R^4$ $-(CH_2)_2-$, $-(CH_2)_3-$, $-(CH_2)_4-$, $-CH_2-CH(R^6)-$, $-CH_2-CHOR^7-CH_2-$;

$R^6$ $C_1$-$C_{12}$-Alkyl;

$R^7$ Wasserstoff, $C_1$-$C_{12}$-Alkyl, $R^6$-C(=O)-, $R^6$-NH-C(=O)-;

n 1 wenn $R^1$ kein Polyalkoholrest ist oder

n 1 bis 8 wenn $R^1$ ein Polyalkoholrest ist

s 0;

u 2 bis 2000;

v 0 bis 2000;

w 0 bis 2000.

Ganz besonders bevorzugt werden als Polyether (b) Polymerisate der allgemeinen Formel I mit einem mittleren Molekulargewicht von 600 bis 20000 (nach dem Zahlenmittel), in der die Variablen unabhängig voneinander folgende Bedeutung haben:

$R^1$ Wasserstoff, $C_1$-$C_6$-Alkyl, $R^6$-C(=O)-, $R^6$-NH-C(=O)-;

$R^5$ Wasserstoff, $C_1$-$C_6$-Alkyl, $R^6$-C(=O)-, $R^6$-NH-C(=O)-;

$R^2$ bis $R^4$ $-(CH_2)_2-$, $-(CH_2)_3-$, $-(CH_2)_4-$, $-CH_2-CH(R^6)-$, $-CH_2-CHOR^7-CH_2-$;

$R^6$ $C_1$-$C_6$-Alkyl;

$R^7$ Wasserstoff, $C_1$-$C_6$-Alkyl, $R^6$-C(=O)-, $R^6$-NH-C(=O)-;

n 1;

s 0;

u 5 bis 500;

v 0 bis 500;

w 0 bis 500.

Als Polyether können jedoch auch Silikonderivate eingesetzt werden. Geeignete Silikonderivate sind die unter dem INCI Namen Dimethicone Copolyole oder Silikontenside bekannten Verbindungen wie zum Beispiel die unter den Markennamen Abil® (der Fa. T. Goldschmidt), Alkasil® (der Fa. Rhône-Poulenc), Silicone Polyol Copolymer® (der Fa Genesee), Belsil® (der Fa. Wacker), Silwet® (der Fa. Witco, Greenwich, CT, USA) oder Dow Corning (der Fa. Dow Corning) erhältlich. Diese beinhalten Verbindungen mit den CAS-Nummern 64365-23-7; 68937-54-2; 68938-54-5; 68937-55-3.

Silikone werden in der Haarkosmetik im allgemeinen zur Verbesserung des Griffs eingesetzt. Die Verwendung von polyetherhaltigen Silikonderivaten als Polyether (b) in den erfindungsgemäßen Polymerisaten kann deshalb zusätzlich zu einer Verbesserung des Griffs der Haare führen.

Bevorzugte Vertreter solcher polyetherhaltigen Silikonderivaten sind solche, die die folgenden Strukturelemente enthalten:

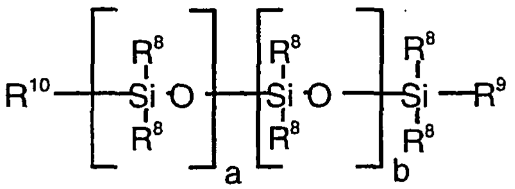 (II)

wobei:

$R^9 = CH_3$ oder

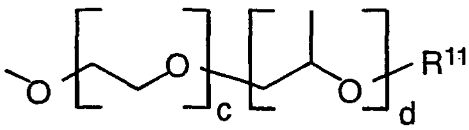

$R^{10} = CH_3$ oder $R^9$

$R^{11}$ = H, $CH_3$,

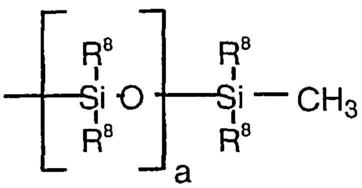

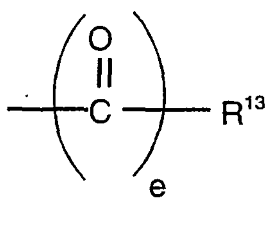

$R^{13}$ ein organischer Rest aus 1 bis 40 Kohlenstoffatomen, der Amino-, Carbonsäure- oder Sulfonatgruppen enthalten kann oder für den Fall e=0, auch das Anion einer anorganischen Säure bedeutet,

und wobei die Reste $R^8$ identisch oder unterschiedlich sein können, und entweder aus der Gruppe der aliphatischen Kohlenwasserstoffe mit 1 bis 20 Kohlenstoffatomen stammen, cyclische aliphatische Kohlenwasserstoffe mit 3 bis 20 C-Atomen sind, aromatischer Natur oder gleich $R^{12}$ sind, wobei:

$R^{12}$ = 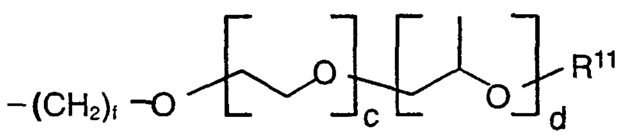

mit der Maßgabe, daß mindestens einer der Reste $R^8$, $R^9$ oder $R^{10}$ ein polyalkylenoxidhaltiger Rest nach obengenannter

Definition ist, und

f eine ganze Zahl von 1 bis 6 ist,

a und b ganze Zahlen derart sind, dass das Molekulargewicht des Polysiloxan-Blocks zwischen 300 und 30000 liegt,

c und d ganze Zahlen zwischen 0 und 50 sein können mit der Maßgabe, dass die Summe aus c und d größer als 0 ist, und e 0 oder 1 ist.

Bevorzugte Reste $R^9$ und $R^{12}$ sind solche, bei denen die Summe aus c+d zwischen 5 und 30 beträgt.

Bevorzugt werden die Gruppen $R^8$ aus der folgenden Gruppe ausgewählt: Methyl, Ethyl, Propyl, Butyl, Isobutyl, Pentyl, Isopentyl, Hexyl, Octyl, Decyl, Dodecyl und Octadecyl, cycloaliphatische Reste, speziell Cyclohexyl, aromatische Gruppen, speziell Phenyl oder Naphthyl, gemischt aromatisch-aliphatische Reste wie Benzyl oder Phenylethyl sowie Tolyl und Xylyl und $R^{12}$.

Besonders geeignete Reste $R^{11}$ sind solche, bei denen im Falle von $R^{11}$ = $-(CO)_e-R^{13}$ $R^{13}$ ein beliebiger Alkyl-, Cycloalkyl oder Arylrest bedeutet, der zwischen 1 und 40 C-Atomen besitzt und der weitere ionogene Gruppen wie $NH_2$, COOH, $SO_3H$ tragen kann.

Bevorzugte anorganische Reste $R^{13}$ sind, für den Fall e=0, Phosphat und Sulfat.

Besonders bevorzugte polyetherhaltige Silikonderivate sind solche der allgemeinen Struktur:

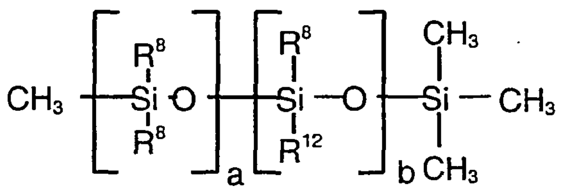

Des weiteren können als Polyether (b) auch Homo- und Copolymerisate aus polyalkylenoxidhaltigen ethylenisch ungesättigten Monomeren wie beispielsweise Polyalkylenoxid(meth)acrylate, Polyalkylenoxidvinylether, Polyalkylenoxid (meth)acrylamide, Polyalkylenoxidallylamide oder Polyalkylenoxidvinylamide verwendet werden. Selbstverständlich können auch Copolymerisate solcher Monomere mit anderen ethylenisch ungesättigten Monomeren eingesetzt werden.

Als polyetherhaltige Verbindungen b) können aber auch Umsetzungsprodukte von Polyethyleniminen mit Alkylenoxiden eingesetzt werden. Als Alkylenoxide werden in diesem Fall bevorzugt Ethylenoxid, Propylenoxid, Butylenoxid und Mischungen aus diesen, besonders bevorzugt Ethylenoxid verwendet. Als Polyethylenimine können Polymere mit zahlenmittleren Molekulargewichten von 300 bis 20000, bevorzugt 500 bis 10000, ganz besonders bevorzugt 500 bis 5000, eingesetzt werden. Das Gewichtsverhältnis zwischen eingesetztem Alkylenoxid und Polyethylenimin liegt im Bereich von 100 : 1 bis 0,1 : 1, bevorzugt im Bereich 50 : 1 bis 0,5 : 1, ganz besonders bevorzugt im Bereich 20 : 1 bis 0,5 : 1.

Bei dem Polyether (b) handelt es sich am meisten bevorzugt um ein Homopolymerisat von Ethylenoxid oder um ein Blockcopolymerisat von Ethylenoxid/Propylenoxid mit einem Molekulargewicht unter 100000 g/mol vorzugsweise unter 20000 g/mol. Die Blöcke können in der Form A-B oder A-B-A oder B-A-B oder anderen Kombinationen vorliegen.

Für die Polymerisation in Gegenwart der Polyether (b) werden als Komponente (a1) radikalisch polymerisierbare Monomere mit mindestens einem quaternärem Stickstoff eingesetzt. Quaternärer Stickstoff meint im Rahmen dieser Erfindung Stickstoff, an den vier organische Reste kovalent gebunden sind. Beispielhaft seien als geeignete Monomere mit einem quaternären Stickstoff zu nennen:

1) Quaternäre Vinylamine der allgemeinen Formeln (IIIa) und (IIIb) sowie deren Salze:

$W-(CH_2)_n-CR^{15}=CHR^{14}$ (IIIa)

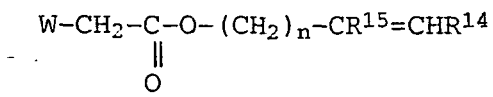 (IIIb)

wobei gilt:

$R^{14}$ und $R^{15}$ sind unabhängig voneinander ausgewählt aus der Gruppe bestehend aus Wasserstoff, $C_1$-$C_8$ linear- oder verzweigtkettige Alkyl, Methoxy, Ethoxy, 2-Hydroxyethoxy, 2-Methoxyethoxy und 2-Ethoxyethyl. Bevorzugt sind Wasserstoff, Methyl oder Ethyl,

n ist 0, 1 oder 2,

W ist

$-\overset{+}{N}(R^{16})_3$ / $x^-$ oder 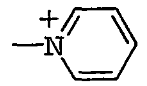 $X^-$

wobei die Reste $R^{16}$ identisch oder verschieden ausgewählt werden können aus der Gruppe bestehend aus $C_1$-$C_{40}$ linear- oder verzweigtkettige Alkylreste, Formyl, $C_1$-$C_{10}$ linear- oder verzweigtkettige Acyl, N,N-Dimethylaminoethyl, 2-Hydroxyethyl, 2-Methoxyethyl, 2-Ethoxyethyl, Hydroxypropyl, Methoxypropyl, Ethoxypropyl oder Benzyl, bevorzugt sind Methyl, Ethyl und n-Propyl, $X^-$ ist ein Anion, bevorzugt ein Anion das kosmetisch verträglich ist. Bevorzugt sind als Anion Acetat, Methylsulfat oder Halogenid wie insbesondere Chlorid oder Bromid.

2) Quaternäre N,N,N-Trialkylaminoalkylacrylate und -methacrylate und quaternäre N,N,N-Trialkylaminoalkylacrylamide und -methacrylamide der allgemeinen Formel (IV) sowie deren Salze.

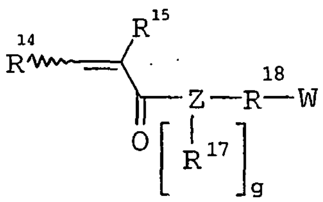

(IV)

wobei $R^{14}$, $R^{15}$ und W die gleiche Bedeutung wie in der allgemeinen Formel IIIa und IIIb haben, und

$R^{17}$ = Wasserstoff oder Methyl,
$R^{18}$ = Alkylen oder Hydroxyalkylen mit 1 bis 24 C-Atomen, optional substituiert durch Alkyl, bevorzugt $C_2H_4$, $C_3H_6$, $C_4H_8$, $CH_2$-$CH(OH)$-$CH_2$
g = 0 oder 1
Z = Stickstoff für g = 1 oder Sauerstoff für g = 0

Die erfindungsgemäß umfassten Amide können unsubstituiert, N-Alkyl oder N-Alkylamino monosubstituiert oder N, N-dialkyl-substituiert oder N,N-dialkylaminodisubstituiert vorliegen, worin die Alkyl- oder Alkylaminogruppen von $C_1$-$C_{40}$ linearen, $C_3$-$C_{40}$ verzweigtkettigen, oder $C_3$-$C_{40}$ carbocyclischen Einheiten abgeleitet sind.
Bevorzugte Monomere der Formel (IV) sind die Salze von N,N,N-Trimethylaminomethyl(meth)acrylat, N,N,N-Triethylaminomethyl(meth)acrylat, N,N,N-Trimethylaminoethyl(meth)acrylat, N,N,N-Triethylaminoethyl-(meth)acrylat, N,N,N-Trimethylaminobutyl(meth)acrylat, N,N,N-Triethylaminobutyl(meth)acrylat, N,N,N-Trimethylaminohexyl (meth)acrylat, N,N,N-Trimethylaminooctyl(meth)acrylat, N,N,N-Trimethylaminododecyl(meth)acrylat.
Ferner sind bevorzugt die Salze von N-[3-(Trimethylamino)-propyl]methacrylamid und N-[3-(Trimethylamino)propyl] acrylamid, N-[3-(Dimethylamino)butyl]methacrylamid, N-[8-(Trimethylamino)octyl]methacrylamid, N-[12-(Trimethylamino)-dodecyl]methacrylamid, N-[3-(Triethylamino)propyl]methacrylamid und N-[3-(Triethylamino)propyl]acrylamid.
Weiterhin bevorzugt sind (Meth)acryloyloxyhydroxypropyltrimethylammoniumchlorid und (Meth)acryloyloxyhydroxypropyltriethylammoniumchlorid.
Ganz besonders bevorzugt sind N,N,N-Trimethylaminoethylmethacrylat und N-[3-(Trimethylamino)propyl]methacrylamid.

3) Quaternäre N-Vinylimidazole der allgemeinen Formel (V) sowie deren Salze,

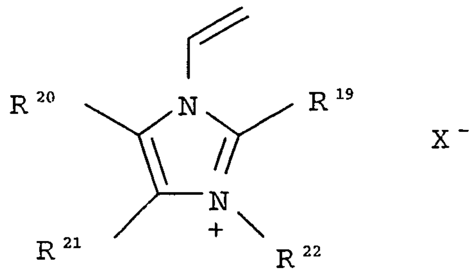

(V)

wobei

$R^{19}$ bis $R^{21}$ unabhängig voneinander für Wasserstoff, $C_1$-$C_4$-Alkyl, $C_1$-$C_4$-Hydroxyalkyl oder Phenyl; und

$R^{22}$ für $C_1$-$C_4$-Alkyl, $C_1$-$C_4$-Hydroxyalkyl oder Phenyl; und

und $X^-$ für ein Anion steht, bevorzugt ein Anion das kosmetisch verträglich ist. Bevorzugt sind als Anion Acetat, Methylsulfat oder Halogenid wie insbesondere Chlorid oder Bromid.

Besonders bevorzugt sind 3-Methyl-1-vinylimidazoliumchlorid, 3-Methyl-1-vinylimidazoliummethylsulfat.

4) Quaternäre Vinylpyridine der allgemeinen Formel (VI) sowie deren Salze, $R^{21}$, $R^{22}$ und $X^-$ die gleiche Bedeutung wie in der allgemeinen Formel V haben.

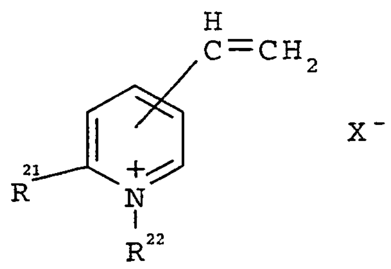

(VI)

5) Weitere geeignete Monomere sind Diallylamine der allgemeinen Formel (VII) sowie deren Salze

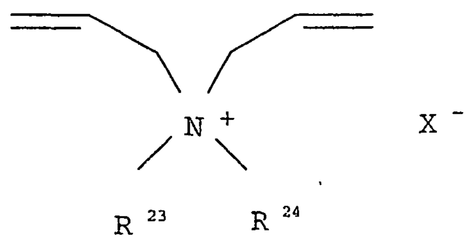

(VII)

wobei $R^{23}$ und $R^{24}$ jeweils und unabhängig voneinander $C_1$- bis $C_{24}$-Alkyl sein können und $X^-$ die gleiche Bedeutung wie in Formel (V) hat. Besonders bevorzugt ist N,N-Dimethyl-N,N-diallylammoniumchlorid.

Selbstverständlich können auch Mischungen der verschiedener Monomeren (a1) aus den oben genannten Gruppen miteinander (und ggf. weiteren Monomeren) polymerisiert werden.

Besonders bevorzugt sind 3-Methyl-1-vinylimidazoliumchlorid, 3-Methyl-1-vinylimidazoliummethylsulfat und N, N-Dimethyl-N,N-diallyl-ammoniumchlorid, sowie Mischungen der vorgenannten.
Ganz besonders bevorzugt sind für (a1) Mischungen aus 3-Methyl-1-vinylimidazoliummethylsulfat und N,N-Dimethyl-N,N-diallylammoniumchlorid.

Direkte Vorprodukte (a2) für die Komponente (a1) umfasst allgemein all solche radikalisch polymerisierbaren Monomere, die durch eine Umsetzung in ein radikalisch polymerisierbares Monomer mit einem quaternären Stickstoff umgesetzt werden können. Für die infolge erhaltenen Verbindungen (a2') gelten die gleichen Definitionen wie sie oben für (a1) gegeben sind. Für die als direkte Vorprodukt für (a1) in Frage kommende Komponente (a2) seien folgende radikalisch polymerisierbare Monomere bevorzugt genannt:

1) Ungesättigte primäre, sekundäre oder tertiäre Amine

Die zu den erfindungsgemäßen Polymerisaten führende Quaternisierung der Amine kann beispielsweise durch Umsetzung der in den Monomeren (a2) enthaltenen Aminogruppen mit Alkylhalogenide mit vorzugsweise 1 bis 24 C-Atomen in der Alkylgruppe realisiert werden. Besonders bevorzugt werden eingesetzt Methylchlorid, Methylbromid, Methyliodid, Ethylchlorid, Ethylbromid, Propylchlorid, Hexylchlorid, Dodecylchlorid, Laurylchlorid und Benzylhalogenide, wie insbesondere Benzylchlorid und Benzylbromid. Andere zur Quaternisierung geeignete Agentien umfassen Dialkylsulfate wie insbesondere Dimethylsulfat oder Diethylsulfat. Eine Quaternisierung basischer Aminogruppen kann ferner mit Alkylenoxiden wie Ethylenoxide oder Propylenoxide in Gegenwart von Säuren realisiert werden. Am meisten werden als Agentien für die Quaternisierung eingesetzt: Methylchlorid, Dimethylsulfat oder Diethylsulfat. Ferner ist eine Umsetzung mit quaternisierten Epichlorhydrin der allgemeinen Formel (XII) möglich (s.u.)

Beispielhaft jedoch nicht einschränkend seien für die als Monomere (a2) geeigneten Amine zu nennen:

a) Aminoalkylacrylate- und -methacrylate und Aminoalkylacryl- und -methacrylamide der allgemeinen Formel (VIII)

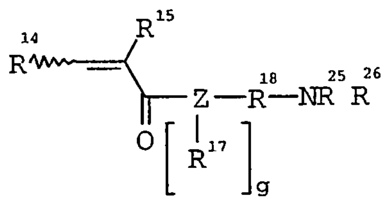

(VIII)

wobei für $R^{14}$ bis $R^{18}$ die für Formel (IV) gegebenen Definitionen gelten und $R^{25}$ bzw. $R^{26}$ jeweils und unabhängig voneinander ausgewählt sind aus der Gruppe bestehend aus Wasserstoff, $C_1$-$C_{40}$ linear- oder verzweigtkettige Alkyl, Formyl, $C_1$-$C_{10}$ linear- oder verzweigtkettige Acyl, N,N-Dimethylaminoethyl, 2-Hydroxyethyl, 2-Methoxyethyl, 2-Ethoxyethyl, Hydroxypropyl, Methoxypropyl, Ethoxypropyl oder Benzyl. Bevorzugt sind Wasserstoff, Methyl, Ethyl, n-Propyl und Benzyl.

Die Amide können unsubstituiert, N-Alkyl oder N-Alkylamino monosubstituiert oder N,N-dialkylsubstituiert oder N,N-dialkylaminodisubstituiert vorliegen, worin die Alkyl- oder Alkylaminogruppen von $C_1$-$C_{40}$ linearen, $C_3$-$C_{40}$ verzweigtkettigen, oder $C_3$-$C_{40}$ carbocyclischen Einheiten abgeleitet sind. Zusätzlich können die Alkylaminogruppen quaternisiert werden.

Bevorzugte Comonomere der Formel (VIII) sind N,N-Dimethylaminomethyl(meth)acrylat, N,N-Diethylaminomethyl-(meth)acrylat, N,N-Dimethylaminoethyl(meth)acrylat, N,N-Diethylaminoethyl(meth)acrylat, N,N-Dimethylaminobutyl(meth)acrylat, N,N-Diethylaminobutyl(meth)acrylat, N,N-Dimethylaminohexyl(meth)acrylat, N,N-dimethylaminooctyl(meth)acrylat, N,N-Dimethylaminododecyl(meth)-acrylat.

Ferner sind bevorzugt N-[3-(dimethylamino)propyl]-methacrylamid, N-[3-(dimethylamino)propyl]acrylamid, N-[3-(Dimethylamino)butyl]methacrylamid, N-[8-(Dimethylamino)octyl]methacrylamid, N-[12-(Dimethylamino) dodecyl]-methacrylamid, N-[3-(Diethylamino)propyl]methacrylamid und N-[3-(Diethylamino)propyl]acrylamide.

Ganz besonders bevorzugt sind N,N-Dimethylaminoethylmethacrylat, N-[3-(dimethylamino)propyl]methacrylamid, N-Methylaminoethylmethacrylat, N-[3-(Methylamino)-propyl]methacrylamid, Aminoethylmethacrylat und N-[3-aminopropyl]methacrylamid.

Bevorzugt erfolgt bei den obengenannten Monomeren eine Quaternisierung unter Verwendung Methylchlorid, Methylsulfat oder Diethylsulfat.

b) N-Vinylimidazole der allgemeinen Formel IX, wobei für $R^{19}$ bis $R^{21}$ unabhängig voneinander die für Formel (V) gegebenen Definitionen gelten.

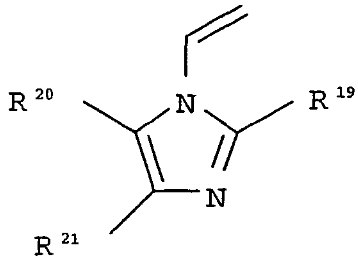

(IX)

Besonders bevorzugt sind N-Vinylimidazol, 1-Vinyl-2-methylvinylimidazol und eine Quaternisierung mit Methylchlorid, Methylsulfat oder Diethylsulfat.

c) Diallylamine der allgemeinen Formel (X)

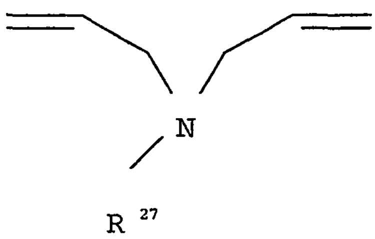

(X)

mit $R^{27}$ = Wasserstoff oder $C_1$- bis $C_{24}$-Alkyl. Besonders bevorzugt ist N,N-Diallylamin und eine Quaternisierung mit Methylchlorid oder Methylsulfat.

d) Ferner kann (a2) ausgewählt sein aus Verbindungen wie 1,3-Divinylimidazolid-2-on oder N-Disubstituierte Vinylaminen der allgemeinen Formel (XI):

$$(R^{28})_2N\text{-}(CH_2)_n\text{-}CR^{15}\text{=}CHR^{14} \qquad \text{(XI)}$$

wobei $R^{14}$, $R^{15}$ und n die gleich Bedeutung wie in den Formeln (IIIa) und (IIIb) haben, und die Reste $R^{28}$ ausgewählt seien können aus der Gruppe bestehend aus Wasserstoff $C_1$-$C_{40}$ linear- oder verzweigtkettige Alkylreste, Formyl, $C_1$-$C_{10}$ linear- oder verzweigtkettige Acyl, N,N-Dimethylaminoethyl, 2-Hydroxyethyl, 2-Methoxyethyl, 2-Ethoxyethyl, Hydroxypropyl, Methoxypropyl, Ethoxypropyl oder Benzyl. Bevorzugt sind Methyl, Ethyl, n-Propyl und Benzyl. Dabei gilt, wenn n=0, dass nicht beide Reste $R^{28}$ gleichzeitig Wasserstoff sind.

2) Ungesättigte Säuren
Quaternäre Amine können durch Umsetzung von Säuren, wie sie beispielsweise durch Verwendung von ungesättigten Säuren wie z.B. Acrylsäure oder Methacrylsäure als Ausgangsverbindung (a2) eingebracht würden, mit einem quaternisierten Epichlorhydrin der allgemeinen Formel (XII) erhalten werden.

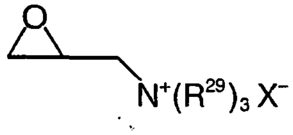

(XII)

Dabei ist $R^{29}$ bevorzugt $C_1$- bis $C_{40}$-Alkyl. Bevorzugt wird 2,3-Epoxypropyl-trimethylammoniumchlorid bzw. 3-Chlor-2-hydroxypropyl-trimethylammoniumchlorid eingesetzt. Die Epoxide der Formel XI können auch in situ durch Umsetzung der entsprechenden Chlorhydrine mit Basen, beispielsweise Natriumhydroxid, erzeugt werden.
Entsprechend können auch Hydroxy- und/oder Aminogruppen enthaltende Monomere (a2) umgesetzt werden. Bevorzugt sind Hydroxylgruppen der Polyvinylalkohol-Einheiten und Vinylamin-Einheiten, entstanden durch Hydrolyse von Vinylformamid.

3) Halogenide radikalisch polymerisierbarer Monomere, wie beispielsweise Halogenalkylacrylate oder Halogenalkylmethacrylate. Bevorzugt sind Chlor-, Brom- oder Iodverbindungen. Beispielhaft jedoch nicht einschränkend sei zu nennen 3-Chlor-2-hydroxypropylacrylat. Die Umsetzung zu quaternären Aminen erfolgt durch Reaktion mit Aminen z.B. Trialkylaminen wie beispielsweise Trimethylamin oder Triethylamin.

Selbstverständlich können auch Mischungen der jeweiligen Monomeren aus der Gruppe (a2) polymerisiert werden. Am meisten bevorzugte Monomere (a2) umfassen N-Vinylimidazol, N,N-Diallylamin und Aminoethylmethacrylat.

Die Umsetzung der Verbindungen (a2) zu quaternären Aminen (infolge a2') kann während oder nach der Reaktion erfolgen. Bei einer nachfolgenden Umsetzung kann das intermediäre Polymerisat zunächst isoliert oder - bevorzugt - unmittelbar umgesetzt werden. Die Umsetzung kann vollständig oder teilweise erfolgen. Dabei werden bevorzugt mindestens 10 %, besonders bevorzugt mindestens 50 %, am meistens bevorzugt mindestens 80 % der Verbindung (a2) zu quaternären Aminen (a2') überführt. Der Anteil der Umsetzung zu quaternären Aminen ist bevorzugt um so höher, je geringer die Wasserlöslichkeit des Monomers (a2) ist. Liegt die Wasserlöslichkeit des Monomers (a2) unter 60 g/l so wird die Umsetzung so durchgeführt, dass das Verhältnis der Summe der Monomere (a2') und ggf. (a1) und (c) zu der Summe der Monomere (a2) und (d) mindestens 2 zu 1 beträgt, bevorzugt 4 zu 1, besonders bevorzugt 10 zu 1.

Die den quaternären Stickstoff enthaltenden polymerisierbaren Monomer (a1) und/oder deren Vorprodukte (a2) können daneben auch in Mischung mit einem oder mehreren, ethylenisch ungesättigten copolymerisierbaren Comonomeren (c) und/oder (d) eingesetzt werden, wobei im Endprodukt das molare Verhältnis der Summe der monomeren Einheiten a1 und/oder a2 und ggf. c zu der Summe der Monomeren d mindestens 2 zu 1 beträgt. Bevorzugt beträgt das Verhältnis mindestens 4 zu 1, ganz besonders bevorzugt mindestens 10 zu 1, am meisten bevorzugt mindestens 20 zu 1.

Als Monomere (c) kommen grundsätzlich alle hydrophilen Monomere mit einer Wasserlöslichkeit oberhalb von 60 g/l bei 25°C in Frage, die mit den Monomeren (a1) und ggf. (a2) und (d) copolymerisierbar sind. Es handelt sich bevorzugt um ethylenisch ungesättigte Monomere. Verschiedene als Monomere (c) geeignete Monomere sind unter anderem beschrieben in Ullmann's Encyclopedia of Industrial Chemistry, Vol. A21, Kapitel "Polyacrylates", S. 157-178, 5. Auflage, 1992, VCH Verlagsgesellschaft mbH, Weinheim, Deutschland.

Der Begriff ethylenisch ungesättigt bedeutet, daß die Monomere zumindest eine radikalisch polymerisierbare Kohlenstoff-Kohlenstoff Doppelbindung besitzen, die mono-, di-, tri- oder tetrasubstituiert sein kann.

Bei den Monomeren (c) handelt es sich vorzugsweise um

i) N-Vinyllactame, bevorzugt mit einem 5 bis 7-Ring, wie z.B. N-Vinylpyrrolidon, N-Vinylpiperidon, N-Vinylcaprolactam,

ii) acyclische N-Vinylcarbonsäureamide, bevorzugt mit 2 bis 6 C-Atomen, wie z.B. N-Vinylformamid, N-Ethyl-N-vinylacetamid oder N-Methyl-N-vinylacetamid,

iii) Hydroxyalkylacrylate, bevorzugt mit 2 bis 6 C-Atomen, wie z.B. 2-Hydroxyethylacrylat, 2-Hydroxyethylmethacrylat, 2-Hydroxypropylmethacrylat, Butandiolmonoacrylat,

iv) ethylenisch ungesättigte Amide, wie beispielsweise Acrylamid oder Methacrylamid,

v) N-Vinylimidazol,

vi) Ungesättigte Säuren, bevorzugt Carbon- oder Sulfonsäuren, wie beispielsweise Acrylsäure, Maleinsäure, Methacrylsäure, 2-Acrylamido-2-methylpropansulfonsäure.

vii) Ungesättigte Amine wie Dimethylaminoethylacrylat, Dimethylaminomethacrylat.

Ferner können beliebige Mischungen verschiedener Monomere (c) eingesetzt werden. Besonders bevorzugte Monomere (c) sind N-Vinyllactame und N-Vinylimidazol. Ganz besonders bevorzugt ist N-Vinylpyrrolidon.

Als Monomere (d) kommen grundsätzlich alle hydrophoben Monomere mit einer Wasserlöslichkeit unterhalb 60 g/l bei 25°C in Frage, die mit den Monomeren (a1) und ggf. (a2) und (c) copolymerisierbar sind. Es handelt sich bevorzugt um ethylenisch ungesättigte Monomere. Verschiedene als Monomere (d) geeignete Monomere sind unter anderem beschrieben in Ullmann's Encyclopedia of Industrial Chemistry, Vol. A21, Kapitel "Polyacrylates", S. 157-178, 5. Auflage, 1992, VCH Verlagsgesellschaft mbH, Weinheim, Deutschland.

Hierbei handelt es sich insbesondere um

1) $C_1$-$C_{10}$-Alkylester monoethylenisch ungesättigter $C_3$-$C_6$-Carbonsäuren, insbesondere die Ester der Acrylsäure

und der Methacrylsäure. Die Ester können abgeleitet sein von $C_1$-$C_{40}$ linearen, $C_3$-$C_{40}$ verzweigtkettigen oder $C_3$-$C_{40}$ carbocyclischen Alkoholen, wie Methanol, Ethanol, n-Propanol, Isopropanol, n-Butanol, Isobutanol, 2-Butanol, tert.-Butanol, n-Pentanol, n-Hexanol, 2-Ethylhexan-1-ol, n-Octanol, n-Decanol, 2-Propylheptan-1-ol, Cyclohexanol, 4-tert.-Butylhexanol oder 2,3,5-Trimethylcyclohexanol. Besonders bevorzugt sind Methylacrylat, Ethylacrylat, Propylacrylat, n-Butylacrylat, iso-Butylacrylat, t-Butylacrylat, 2-Ethylhexylacrylat, Decylacrylat, Methylmethacrylat, Ethylmethacrylat, Propylmethacrylat, n-Butylmethacrylat, iso-Butylmethacrylat, t-Butylmethacrylat, 2-Ethylhexylmethacrylat, Decylmethacrylat, Methylethacrylat, Ethylethacrylat, n-Butylethacrylat, iso-Butylethacrylat, t-Butylethacrylat, 2-Ethylhexylethacrylat, Decylethacrylat, Stearylacrylat, Stearyl(meth)acrylat. Ester können auch von mehrfachfunktionellen Alkoholen mit 2 bis etwa 8 Hydroxylgruppen abgeleitet sein, so lange sie die Löslichkeitsanforderungen für Monomere (d) genügen. Beispielhaft seien Ester von Ethylenglycol, Hexylenglycol, Glycerin und 1,2,6-Hexantriol, von Aminoalkoholen oder von Alkoholethern wie Methoxyethanol und Ethoxyethanol, (Alkyl)Polyethylenglykolen, (Alkyl)Polypropylenglykolen oder ethoxylierten Fettalkoholen, beispielsweise $C_{12}$-$C_{24}$-Fettalkoholen umgesetzt mit 1 bis 200 Ethylenoxid-Einheiten zu nennen.

2) Di-$C_1$-$C_{10}$-alkylester ethylenisch ungesättigter Dicarbonsäuren wie Maleinsäure, Fumarsäure oder Itaconsäure mit den oben unter 1) genannten $C_1$-$C_{10}$-Alkanolen oder $C_5$-$C_{10}$-Cycloalkanolen, z.B. Maleinsäuredimethylester oder Maleinsäuredin-butylester.

3) Kohlenwasserstoffe mit mindestens einer radikalisch polymerisierbaren Kohlenstoff-Kohlenstoff Doppelbindung, bevorzugt Styrol, alpha-Methylstyrol, tert.-Butylstyrol, Butadien, Isopren, Cyclohexadien, Ethylen, Propylen, 1-Buten, 2-Buten, Isobutylen, Vinyltoluol, sowie Mischungen dieser Monomere. Besonders bevorzugt sind vinylaromatische Verbindungen wie Styrol und $\alpha$-Methylstyrol, die gegebenenfalls am aromatischen Ring einen oder mehrere Substituenten aufweisen können, die bevorzugt ausgewählt sind unter $C_1$-$C_4$-Alkyl, Halogenatomen, insbesondere Chlor, und/oder Hydroxylgruppen.

4) Vinyl-, Vinyliden- oder Allylhalogenide, bevorzugt Vinylchlorid, Vinylidenchlorid und Allylchlorid.

5) Vinyl-, Allyl- und Methallylester von $C_1$-$C_{40}$ linearen, $C_3$-$C_{40}$ verzweigtkettigen oder $C_3$-$C_{40}$ carbocyclische Carbonsäuren aliphatischer, gesättigter oder ungesättigter Natur. Als Carbonsäuren bevorzugt sind z.B. Ameisensäure, Essigsäure, Propionsäure, Buttersäure, Valeriansäure, Isovaleriansäure, Capronsäure, Caprylsäure, Caprinsäure, Undecylensäure, Laurinsäure, Myristinsäure, Palmitinsäure, Palmitoleinsäure, Stearinsäure, Ölsäure, Arachinsäure, Behensäure, Lignocerinsäure, Cerotinsäure sowie Melissensäure. Bevorzugt werden Vinylester der oben genannten $C_1$-$C_{12}$-Carbonsäuren, insbesondere der $C_1$-$C_6$-Carbonsäuren, verwendet. Ganz besonders bevorzugt sind Vinylacetat, Vinylpropionat, Vinylbutyrat, Vinylvalerat, Vinylhexanoat, Vinyl-2-ethylhexanoat, Vinyldecanoat, Vinyllaurat und Vinylstearat sowie die entsprechenden Allyl- und Methallylester. Am meisten bevorzugt ist Vinylacetat.

6) Vinyl-, Allyl- und Methallylether linearer oder verzweigter, aliphatischer Alkohole mit 2 bis 20 C-Atomen, z.B. Vinylmethylether, Vinylethylether, Vinyldodecylether, Vinylhexadecylether und Vinylstearylether.

7) Monoethylenisch ungesättigter Monocarbonsäuren, sofern sie eine Löslichkeit in Wasser von unter 60 g/l bei 25°C aufweisen, wie beispielsweise Acrylamidoglycolsäure, Fumarsäure oder Crotonsäure.

Ferner können beliebige Mischungen verschiedener Monomere (d) eingesetzt werden. Besonders bevorzugte Monomere (d) sind Vinylacetat, Methylmethacrylat, Methylacrylat und Ethylacrylat.

Zusätzlich zu den oben genannten Comonomeren können als Comonomere (c) oder (d) sogenannte Makromonomere wie zum Beispiel silikonhaltige Makromonomere mit ein oder mehreren radikalisch polymerisierbaren Gruppen oder Alkyloxazolinmakromonomere eingesetzt werden, wie sie zum Beispiel in der EP 408 311 beschrieben sind. Des weiteren können fluorhaltige Monomere, wie sie beispielsweise in der EP 558423 beschrieben sind, vernetzend wirkende oder das Molekulargewicht regelnde Verbindungen in Kombination oder alleine eingesetzt werden. Die Zuordnung zu den Gruppen (c) oder (d) erfolgt entsprechend ihrer Löslichkeit.

Dem Fachmann ist bewusst, dass ein Monomer (a2) in die Gruppe der Monomere (c) oder (d) fallen kann, solange (a2) nicht in ein quaternäres Amin (a2') umgesetzt wurde.

Die basischen Monomere (c) oder (d) können auch kationisiert werden, indem sie mit Mineralsäuren, wie z.B. Schwefelsäure, Chlorwasserstoffsäure, Bromwasserstoffsäure, Iodwasserstoffsäure, Phosphorsäure oder Salpetersäure, oder mit organischen Säuren, wie z.B. Ameisensäure, Essigsäure, Milchsäure, oder Citronensäure, neutralisiert werden.

Als Regler können die üblichen dem Fachmann bekannten Verbindungen, wie zum Beispiel Schwefelverbindungen (z.B.: Mercaptoethanol, 2-Ethylhexylthioglykolat, Thioglykolsäure oder Dodecylmercaptan), sowie Tribromchlormethan oder andere Verbindungen, die regelnd auf das Molekulargewicht der erhaltenen Polymerisate wirken, verwendet werden.

Es können gegebenenfalls auch thiolgruppenhaltige Silikonverbindungen eingesetzt werden.

Bevorzugt werden silikonfreie Regler eingesetzt.

Als Monomere (a1), (a2), (c) oder (d) können auch vernetzende Monomere eingesetzt werden, beispielsweise Verbindungen mit mindestens zwei ethylenisch ungesättigten Doppelbindungen, wie zum Beispiel Ester von ethylenisch ungesättigten Carbonsäuren, wie Acrylsäure oder Methacrylsäure und mehrwertigen Alkoholen, Ether von mindestens zweiwertigen Alkoholen, wie zum Beispiel Vinylether oder Allylether. Außerdem geeignet sind geradkettige oder verzweigte, lineare oder cyclische aliphatische oder aromatische Kohlenwasserstoffe, die über mindestens zwei Doppelbindungen verfügen, welche bei den aliphatischen Kohlenwasserstoffen nicht konjugiert sein dürfen. Ferner geeignet sind Amide der Acryl- und Methacrylsäure und N-Allylamine von mindestens zweiwertigen Aminen, wie zum Beispiel 1,2-Diaminoethan, 1,3-Diaminopropan. Ferner sind Triallylamin, N-Vinylverbindungen von Harnstoffderivaten, mindestens zweiwertigen Amiden, Cyanuraten oder Urethanen. Weitere geeignete Vernetzer sind Divinyldioxan, Tetraallylsilan oder Tetravinylsilan.

Besonders bevorzugte Vernetzer sind beispielsweise Methylenbisacrylamid, Triallylamin und Triallylammoniumsalze, Divinylimidazol, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind.

Ein ganz besonders bevorzugter Vernetzer ist Divinylethylenharnstoff.

Die vernetzenden Monomeren werden in einer Menge von kleiner als 5 Gew.-%, bezogen auf die Summe der Ausgangsstoffe a) bis d), eingesetzt. Besonders bevorzugt werden weniger als 3 Gew.-% und ganz besonders bevorzugt weniger als 1 Gew.-% vernetzendes Monomer eingesetzt

Bei der Polymerisation zur Herstellung der erfindungsgemäßen Polymerisate können gegebenenfalls auch andere Polymere, wie zum Beispiel Polyamide, Polyurethane, Polyester, Homo- und Copolymere von ethylenisch ungesättigten Monomeren, zugegen sein. Beispiele für solche zum Teil auch in der Kosmetik eingesetzten Polymeren sind die unter den Handelsnamen bekannten Polymere Amerhold™, Ultrahold™, Ultrahold Strong™, Luviflex™ VBM, Luvimer™, Acronal™, Acudyne™, Stepanhold™, Lovocryl™, Versatyl™, Amphomer™ oder Eastma AQ™. Ferner können kationische Guarderivate wie Guarhydroxypropyltrimoniumchlorid (INCI) verwendet werden.

Die erfindungsgemäßen Comonomere (c) und/oder (d) können, sofern sie ionisierbare Gruppen enthalten, vor oder nach der Polymerisation, zum Teil oder vollständig mit Säuren oder Basen neutralisiert werden, um so zum Beispiel die Wasserlöslichkeit oder -dispergierbarkeit auf ein gewünschtes Maß einzustellen.

Als Neutralisationsmittel für Säuregruppen tragende Monomere können zum Beispiel Mineralbasen wie Natriumcarbonat, Alkalihydroxide sowie Ammoniak, organische Basen wie Aminoalkohole speziell 2-Amino-2-Methyl-1-Propanol, Monoethanolamin, Diethanolamin, Triethanolamin, Triisopropanolamin, Tri[(2-hydroxy)1-Propyl]amin, 2-Amino-2-Methyl-1,3-Propandiol, 2-Amino-2-hydroxymethyl-1,3-Propandiol sowie Diamine, wie zum Beispiel Lysin, verwendet werden.

Zur Herstellung der Polymerisate können die Monomeren der Komponente a1) in Gegenwart der Polyether sowohl mit Hilfe von Radikale bildenden Initiatoren als auch durch Einwirkung energiereicher Strahlung, worunter auch die Einwirkung energiereicher Elektronen verstanden werden soll, polymerisiert werden.

Als Initiatoren für die radikalische Polymerisation können die hierfür üblichen Peroxo- und/oder Azo-Verbindungen eingesetzt werden, beispielsweise Alkali- oder Ammoniumperoxidisulfate, Diacetylperoxid, Dibenzoylperoxid, Succinylperoxid, Di-tert.-butylperoxid, tert.-Butylperbenzoat, tert.-Butylperpivalat, tert.-Butylpermaleinat, Cumolhydroperoxid, Diisopropylperoxidicarbamat, Bis-(o-toluoyl)-peroxid, Didecanoylperoxid, Dioctanoylperoxid, Dilauroylperoxid, tert.-Butylperisobutyrat, tert.-Butylperacetat, Di-tert.-Amylperoxid, tert.-Butylhydroperoxid, Azo-bis-isobutyronitril, Azo-bis-(2-amidonopropan)dihydrochlorid oder 2-2'-Azo-bis-(2-methyl-butyronitril). Geeignet sind auch Initiatormischungen oder Redox-Initiator-Systeme, wie z.B. Ascorbinsäure/Eisen(II)sulfat/Natriumperoxodisulfat, tert.-Butylhydroperoxid/Natriumdisulfit, tert.-Butylhydroperoxid/Natriumhydroxymethansulfinat.

Bevorzugt werden organische Peroxide eingesetzt.

Die verwendeten Mengen an Initiator bzw. Initiatorgemischen bezogen auf die eingesetzten radikalisch polymerisierbaren Monomeren (a1, a2, c, d) liegen zwischen 0,01 und 100 Gew.-%, vorzugsweise zwischen 0,1 und 40 Gew.-%, besonders bevorzugt zwischen 1 und 15 Gew.-%.

Die Polymerisation erfolgt im Temperaturbereich von 40 bis 200°C, bevorzugt im Bereich von 50 bis 140°C, besonders bevorzugt im Bereich von 60 bis 110°C. Sie wird üblicherweise unter atmosphärischem Druck durchgeführt, kann jedoch auch unter vermindertem oder erhöhtem Druck, vorzugsweise zwischen 1 und 5 bar, ablaufen. Bevorzugt wird die Reaktionstemperatur so gewählt, dass sie zumindest der Schmelztemperatur der polyetherhaltige Verbindung (b) unter den jeweiligen Reaktionsbedingungen entspricht, so dass die Reaktion in einer Schmelze von (b) durchgeführt werden kann.

Die Polymerisation kann beispielsweise als Lösungspolymerisation, Polymerisation in Substanz, Emulsionspolymerisation, umgekehrte Emulsionspolymerisation, Suspensionspolymerisation, umgekehrte Suspensionspolymerisation oder Fällungspolymerisation durchgeführt werden, ohne daß die verwendbaren Methoden darauf beschränkt sind. Bevorzugt ist die Polymerisation in Substanz, wobei die Polymerisation von a1 und ggf. a2, c und/oder d in Gegenwart von b durchgeführt wird. Dabei ist der Gehalt an Wasser im Reaktionsgemisch während der Polymerisation kleiner als 20 Gew.-%, bevorzugt kleiner als 15 %, besonders bevorzugt kleiner als 10 Gew.-%, am meisten bevorzugt kleiner als 5 Gew.-%. Bevorzugt wird unter im wesentlichen wasserfreien Bedingungen gearbeitet und eine Polymerisation in Substanz durchgeführt. "Im wesentlichen wasserfrei" bedeutet in diesem Zusammenhang, dass neben dem in den Ausgangsprodukten enthaltenen Wasser kein zusätzliches Wasser in das Reaktionsgemisch eingebracht wird. "Während der Polymerisation" bedeutet in diesem Zusammenhang, dass die radikalische Polymerisation der radikalisch polymerisierbaren Monomere noch nicht abgeschlossen ist. Dabei wird eine Polymerisation solange als nicht abgeschlossenen betrachtet, solange der Gehalt an Restmonomeren im Vergleich zu der Ausgangsmenge an Monomeren noch größer ist als 50 %, bevorzugt größer als 30 %, besonders bevorzugt größer als 10 %, ganz besonders bevorzugt größer als 5 %, am meisten bevorzugt größer als 2 %. Nach Abschluss der Polymerisation gemäß obiger Definition ist es möglich dem Reaktionsgemisch Wasser auch in höheren Mengen (d.h. zu mehr als 20 Gew.-% am Reaktionsgemisch) zuzusetzen.

Bei der besonders bevorzugten Polymerisation in Substanz kann man so vorgehen, dass man mindestens ein Monomer der Gruppe (a1) und/oder (a2) und/oder eventuell weiteren Comonomeren der Gruppen (c) und/oder (d) in der polyetherhaltigen Verbindung (b) löst und nach Zugabe eines Polymerisationsinitiators die Mischung auspolymerisiert. Die Polymerisation kann auch halbkontinuierlich durchgeführt werden, indem man zunächst einen Teil, z.B. 10 % des zu polymerisierenden Gemisches aus der polyetherhaltigen Verbindung (b), mindestens einem Monomeren der Gruppe (a1) und/oder (a2), eventuell weiteren Comonomeren der Gruppen (c) und/oder (d) und Initiator vorlegt, das Gemisch auf Polymerisationstemperatur erhitzt und nach dem Anspringen der Polymerisation den Rest der zu polymerisierenden Mischung nach Fortschritt der Polymerisation zugibt. Die Polymerisate können - am meisten bevorzugt - auch dadurch erhalten werden, daß man die polyetherhaltigen Verbindungen der Gruppe (b) in einem Reaktor vorlegt, auf die Polymerisationstemperatur erwärmt und mindestens ein Monomer der Gruppe (a1) und/oder (a2), eventuell weiteren Comonomeren der Gruppen (c) und/oder (d) und Polymerisationsinitiator entweder auf einmal, absatzweise oder vorzugsweise kontinuierlich zufügt und polymerisiert.

Für die Polymerisation können Emulgatoren zugesetzt werden.
Als Emulgatoren verwendet man beispielsweise ionische oder nichtionische Tenside, deren HLB-Wert im Bereich von 3 bis 18 liegt. Zur Definition des HLB-Werts wird auf die Veröffentlichung von Griffin WC (1954) J Soc Cosmetic Chem Band 5, S.249 hingewiesen. Die Menge an Tensiden, bezogen auf das Polymerisat, beträgt 0 bis 10 Gew.-%. Vorzugsweise werden für die Polymerisation keine Tenside zugesetzt.

Die Polymerisationen zur Herstellung der erfindungsgemäßen Polymerisate können in Gegenwart mindestens eines nicht-wässrigen, organischen Lösungsmittels oder in Mischungen aus mindestens einem organischen Lösemittel und Wasser durchgeführt werden. Bevorzugt verwendet man pro 100 Gew.-Teile der Summe der Edukte (a1 und/oder a2, b, und gegebenenfalls c und d) 5 bis 2000, vorzugsweise 10 bis 500 Gew.-Teile des organischen Lösemittels oder des Lösemittelgemisches. Geeignete Lösemittel sind beispielsweise Alkohole, wie Methanol, Ethanol, n-Propanol, Isopropanol, n-Butanol, sek.-Butanol, tert.-Butanol, n-Hexanol und Cyclohexanol sowie Glykole, wie Ethylenglykol, Propylenglykol und Butylenglykol sowie die Methyl- oder Ethylether der zweiwertigen Alkohole, Diethylenglykol, Triethylenglykol, Glycerin und Dioxan.

Bevorzugt sind Polymerisate, die erhältlich sind durch radikalische Polymerisation von

1) 3 bis 30 Gew.-% mindestens eines Monomers mit quaternären Aminogruppen (a1) und

2) 0 bis 15 Gew.-% eines oder mehreren weiteren copolymerisierbaren Monomeren (c) und

3) 0 bis 15 Gew.-% eines oder mehreren weiteren copolymerisierbaren Monomeren (d) in Gegenwart von

4) 70 bis 97 Gew.-% mindestens einer polyetherhaltigen Verbindung,

wobei sich für eine definierte Polymerisation die Gew.-% der einzelnen Komponenten a1 - d auf jeweils 100 Gew.-% addieren.

Besonders bevorzugt sind Polymerisate, die erhältlich sind durch radikalische Polymerisation von

1) 4 bis 12 Gew.-% mindestens eines Monomers mit quaternären Aminogruppen (a1) und

2) 0 Gew.-% eines oder mehreren weiteren copolymerisierbaren Monomeren (c)

3) 0 Gew.-% eines oder mehreren weiteren copolymerisierbaren Monomeren in (d) Gegenwart von

4) 88 bis 96 Gew.-% mindestens einer polyetherhaltigen Verbindung,

wobei sich für eine definierte Polymerisation die Gew.-% der einzelnen Komponenten a1 und b auf jeweils 100 Gew.-% addieren.

Die K-Werte der Polymerisate sollen im Bereich von 10 bis 300, bevorzugt 11 bis 100, besonders bevorzugt 15 bis 60 liegen. Der jeweils gewünschte K-Wert läßt sich in an sich bekannter Weise durch die Zusammensetzung der Einsatzstoffe einstellen. Die K-Werte werden bestimmt nach Fikentscher, Cellulosechemie, Bd. 13, S. 58 bis 64, und 71 bis 74 (1932) in N-Methylpyrrolidon bei 25°C und Polymerkonzentrationen, die je nach K-Wert-Bereich zwischen 0,1 Gew.-% und 5 Gew.-% liegen. Auch andere Lösemittel anstelle von N-Methylpyrrolidon können verwendet werden. Besonders bevorzugt ist Ethanol. Die oben angegebenen Grenzen beziehen sich bevorzugt auf die Bestimmung 1 %iger Polymerlösungen in Ethanol.

Nach der Umsetzung können die Polymerlösungen zur Entfernung von beispielsweise Restmonomeren wasserdampfdestilliert werden. Nach der Wasserdampfdestillation erhält man je nach Menge der quaternisierten Aminogruppen und der Art der Polyether (b) wässrige Lösungen oder Dispersionen. Der Erhalt wässriger Lösungen ist bevorzugt. Vorteilhafterweise werden den erfindungsgemäßen Polymerisaten aus Gründen einer besseren Handhabbarkeit nach Beendigung der Polymerisation Wasser zugesetzt. Dabei beträgt der Gehalt an Polymerisat bevorzugt 20 bis 60 Gew.-%.

Die Polymerisate können durch verschiedene Trocknungsverfahren wie z.B. Sprühtrocknung, Fluidized Spray Drying, Walzentrocknung oder Gefriertrocknung in Pulverform überführt werden. Als Trocknungsverfahren wird bevorzugt die Sprühtrocknung eingesetzt. Aus dem so erhaltenen Polymer-Trockenpulver läßt sich durch Lösen bzw. Redispergieren in Wasser erneut eine wässrige Lösung bzw. Dispersion herstellen. Die Überführung in Pulverform hat den Vorteil einer besseren Lagerfähigkeit, einer einfacheren Transportmöglichkeit sowie eine geringere Neigung für Keimbefall.

Die erfindungsgemäßen wasserlöslichen oder wasserdispergierbaren polyetherhaltigen Polymerisate können vorteilhaft zu zahlreichen Zwecken verwendet werden. Beispielhaft seien genannt eine Verwendung

a) in kosmetischen Zubereitungen

b) in Arzneimitteln und anderen therapeutisch-medizinischen Zubereitungen

c) in Formulierungen für Reinigungsmittel, Desinfektionsmitteln oder Geschirrspülmittel.

d) in Textil- und/oder Teppichpflegemitteln z.B. in Weichspülern oder in Wasch- und Pflegemitteln

e) als Stabilisatoren für beispielsweise Dispersionen z.B. bei der Durchführung von Polymerisationen in wässriger Lösung oder Emulsion

f) als Stabilisator für die Herstellung fotografischer Emulsionen

g) als Flockungsmittel beispielsweise bei der Abwasserbehandlung

h) als Hilfsmittel zur Papierherstellung insbesondere zur Herstellung von Papieren für die Verwendung in "Ink Jet" Verfahren

i) in der Färbeindustrie als Zusatz zu Farben oder Tinten

j) als Feuchthaltemittel oder Gelbildner

k) als Gelantineersatzstoff

l) als Verdickungsmittel

m) als Dehydrierungsmittel

Die erfindungsgemäßen wasserlöslichen oder wasserdispergierbaren Polyalkylenoxid- bzw. Polyglycerin-haltigen Polymerisate eignen sich insbesondere hervorragend zur Verwendung in kosmetischen Formulierungen, insbesondere haarkosmetischen Formulierungen.

Der Begriff der kosmetischen Formulierungen ist breit zu verstehen und meint all solche Zubereitungen, die sich zum Auftragen auf Haut und/oder Haare und/oder Nägel eignen und einen anderen als einen ausschließlich medizinisch-therapeutischen Zweck verfolgen.

Haarkosmetische Formulierungen umfasst insbesondere Stylingmittel und/oder Konditioniermittel in haarkosmetischen Zubereitungen wie Haarkuren, Haarschäume (engl. Mousses), (Haar)gelen oder Haarsprays, Haarlotionen, Haarspülungen, Haarshampoos, Haaremulsionen, Spitzenfluids, Egalisierungsmittel für Dauerwellen, Haarfärbe- und -bleichmittel, "Hot-Oil-Treatment"-Präparate, Conditioner, Festigerlotionen oder Haarsprays. Je nach Anwendungsgebiet können die haarkosmetischen Zubereitungen als (Aerosol-)Spray, (Aerosol-)Schaum, Gel, Gelspray, Creme, Lotion oder Wachs appliziert werden.

Weitere kosmetische Zubereitungen umfassen beispielsweise Conditioner für die Haut, z.B. in Haut- bzw. Körperpflegemitteln, Schaum- und Duschbädern. Ferner umfasst ist die Verwendung in der Mundhygiene und anderen Hygiene-Formulierungen, in Anti-Aknemitteln, in Sonnenschutzmitteln, in Bräunungsmitteln, in pigmenthaltigen Formulierungen der dekorativen Kosmetik, in Antifaltenmitteln, in Hautstraffungsmitteln, in Deodorantien.

Die erfindungsgemäßen haarkosmetischen Formulierungen enthalten in einer bevorzugten Ausführungsform

a) 0,05 - 20 Gew.-% des erfindungsgemäßen Polymerisates
b) 20 - 99,95 Gew.-% Wasser und/oder Alkohol
c) 0 - 79,5 Gew.-% weitere Bestandteile

Unter Alkohol sind alle in der Kosmetik üblichen Alkohole zu verstehen, z.B. Ethanol, Isopropanol, n-Propanol.

Unter weiteren Bestandteilen sind die in der Kosmetik üblichen Zusätze zu verstehen, beispielsweise Treibmittel, Entschäumer, grenzflächenaktive Verbindungen, d.h. Tenside, Emulgatoren, Schaumbildner und Solubilisatoren. Die eingesetzten grenzflächenaktiven Verbindungen können anionisch, kationisch, amphoter oder neutral sein. Weitere übliche Bestandteile können ferner sein z.B. Konservierungsmittel, Parfümöle, Trübungsmittel, Wirkstoffe, UV-Filter, Pflegestoffe wie Panthenol, Collagen, Vitamine, Eiweißhydrolysate, Alpha- und Beta-Hydroxycarbonsäuren, Eiweißhydrolysate, Stabilisatoren, pH-Wert-Regulatoren, Farbstoffe, Viskositätsregulierer, Gelbildner, Farbstoffe, Salze, Feuchthaltemittel, Rückfetter, Komplexbildner und weitere übliche Additive.

Weiterhin zählen hierzu alle in der Kosmetik bekannten Styling- und Conditionerpolymere, die in Kombination mit den erfindungsgemäßen Polymerisaten eingesetzt werden können, falls ganz spezielle Eigenschaften eingestellt werden sollen.

Als herkömmliche Haarkosmetik-Polymere eignen sich beispielsweise anionische Polymere. Solche anionischen Polymere sind Homo- und Copolymerisate von Acrylsäure und Methacrylsäure oder deren Salze, Copolymere von Acrylsäure und Acrylamid und deren Salze; Natriumsalze von Polyhydroxycarbonsäuren, wasserlösliche oder wasserdispergierbare Polyester, Polyurethane (Luviset® P.U.R.) und Polyharnstoffe. Besonders geeignete Polymere sind Copolymere aus t-Butylacrylat, Ethylacrylat, Methacrylsäure (z.B. Luvimer® 100P), Copolymere aus N-tert.-Butylacrylamid, Ethylacrylat, Acrylsäure (Ultrahold® 8, Strong), Copolymere aus Vinylacetat, Crotonsäure und gegebenenfalls weiteren Vinylestern (z.B. Luviset® Marken), Maleinsäureanhydridcopolymere, ggf. mit Alkoholen umgesetzt, anionische Polysiloxane, z.B. carboxyfunktionelle, Copolymere aus Vinylpyrrolidon, t-Butylacrylat, Methacrylsäure (z.B Luviskol® VBM) .

Weiterhin umfasst die Gruppe der zur Kombination mit den erfindungsgemäßen Polymerisaten geeigneten Polymere beispielhaft Balance® CR (National Starch; Acrylatcopolymer), Balance® 0/55 (National Starch; Acrylatcopolymer), Balance® 47 (National Starch; Octylacrylamid/Acrylat/Butylaminoethylmethacrylate-Copolymer), Aquaflex® FX 64 (ISP; Isobutylen/Ethylmaleimid/Hydroxyethylmaleimid-Copolymer), Aquaflex® SF-40 (ISP/National Starch; VP/Vinyl Caprolactam/DMAPA Acrylatcopolymer), Allianz® LT-120 (ISP/Rohm & Haas; Acrylat/C1-2 Succinat/Hydroxyacrylat-Copolymer), Aquarez® HS (Eastman; Polyester-1), Diaformer® Z-400 (Clariant; Methacryloyl ethylbetain/Methacrylat-Copolymer), Diaformer® Z-711 (Clariant; Methacryloylethyl N-oxid/Methacrylat-Copolymer), Diaformer® Z-712 (Clariant; Methacryloylethyl N-oxide/Methacrylat-Copolymer), Omnirez® 2000 (ISP; Monoethylester von Poly(Methylvinylether/Maleinsäure) in Ethanol), Amphomer® HC (National Starch; Acrylat/ Octylacrylamid-Copolymer), Amphomer® 28-4910 (National Starch; Octyl-acrylamid/Acrylat/Butylaminoethylmethacrylat-Copolymer), Advantage® HC 37 (ISP; Terpolymer aus Vinyl caprolactam/Vinylpyrrolidon/Dimethylaminoethylmethacrylat), Acudyne® 258 (Rohm & Haas; Acrylat/ Hydroxyesteracrylat-Copolymer), Luviset® PUR (BASF, Polyurethane-1), Luviflex® Silk (BASF), Eastman® AQ48 (Eastman).

Ganz besonders bevorzugt werden als anionische Polymere Acrylate mit einer Säurezahl größer gleich 120 und Copolymere aus t-Butylacrylat, Ethylacrylat, Methacrylsäure.

Weitere geeignete Haarkosmetik-Polymere sind kationische Polymere mit der Bezeichnung Polyquaternium nach INCI, z.B. Copolymere aus Vinylpyrrolidon/N-Vinylimidazoliumsalzen (Luviquat® FC, Luviquat® HM, Luvicluat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam N-Vinylpyrrolidon/N-Vinylimidazoliumsalzen (Luviquat® Hold);

kationische Cellulosederivate (Polyquaternium-4 und -10), Acrylamidcopolymere (Polyquaternium-7).

Ferner können kationische Guarderivate wie Guarhydroxypropyltrimoniumchlorid (INCI) verwendet werden.

Als weitere Haarkosmetik-Polymere sind auch neutrale Polymere geeignet wie Polyvinylpyrrolidone, Copolymere aus N-Vinylpyrrolidon und Vinylacetat und/oder Vinylpropionat, Polysiloxane, Polyvinylcaprolactam und Copolymere mit N-Vinylpyrrolidon, Polyethylenimine und deren Salze, Polyvinylamine und deren Salze, Cellulosederivate, Polyasparaginsäuresalze und Derivate.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze oder Dimethicon Copolyole (CTFA) und aminofunktionelle Silikonverbindungen wie Amodimethicone (CTFA).

Die erfindungsgemäßen Polymerisate eignen sich insbesondere als Festigungsmittel in Haarstyling-Zubereitungen, insbesondere Haarsprays (Aerosolsprays und Pumpsprays ohne Treibgas) und Haarschäume (Aerosolschäume und Pumpschäume ohne Treibgas). In einer bevorzugten Ausführungsform enthalten diese Zubereitungen

a) 0,1 - 10 Gew.-% des erfindungsgemäßen Polymerisates
b) 20 - 99,9 Gew.-% Wasser und/oder Alkohol
c) 0-70 Gew.-% eines Treibmittel
d) 0-20 Gew.-% weitere Bestandteile

Treibmittel sind die für Haarsprays oder Aerosolschäume üblich verwendeten Treibmittel. Bevorzugt sind Gemische aus Propan/ Butan, Pentan, Dimethylether, 1,1-Difluorethan (HFC-152 a), Kohlendioxid, Stickstoff oder Druckluft.

Eine erfindungsgemäß bevorzugte Formulierung für Aerosolhaarschäume enthält

a) 0,1 - 10 Gew.-% des erfindungsgemäßen Polymerisates
b) 55 - 94,8 Gew.-% Wasser und/oder Alkohol
c) 5 - 20 Gew.-% eines Treibmittel
d) 0,1- 5 Gew.-% eines Emulgators
e) 0 - 10 Gew.-% weitere Bestandteile

Als Emulgatoren können alle in Haarschäumen üblicherweise eingesetzten Emulgatoren verwendet werden. Geeignete Emulgatoren können nichtionisch, kationisch bzw. anionisch oder amphoter sein.

Beispiele für nichtionische Emulgatoren (INCI-Nomenklatur) sind Laurethe, z.B. Laureth-4; Cethethe, z.B. Ceteth-1, Polyethylenglycolcetylether; Ceteharethe, z.B. Cetheareth-25, Polyglycolfettsäureglyceride, hydroxyliertes Lecithin, Lactylester von Fettsäuren, Alkylpolyglycoside.

Beispiele für kationische Emulgatoren sind Cetyldimethyl-2-hydroxyethylammoniumdihydrogenphosphat, Cetyltrimoniumchlorid, Cetyltrimmoniumbromid, Cocotrimoniummethylsulfat, Quaternium-1 bis x (INCI).

Anionische Emulgatoren können beispielsweise ausgewählt werden aus der Gruppe der Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Eine erfindungsgemäß für Styling-Gele geeignete Zubereitung kann beispielsweise wie folgt zusammengesetzt sein:

a) 0,1 - 10 Gew.-% des erfindungsgemäßen Polymerisates
b) 60 - 99,85 Gew.-% Wasser und/oder Alkohol
c) 0,05 - 10 Gew.-% eines Gelbildners
d) 0 - 20 Gew.-% weitere Bestandteile

Als Gelbildner können alle in der Kosmetik üblichen Gelbildner eingesetzt werden. Hierzu zählen leicht vernetzte Polyacrylsäure, beispielsweise Carbomer (INCI), Cellulosederivate, z.B. Hydroxypropylcellulose, Hydroxyethylcellulose, kationisch modifizierte Cellulosen, Polysaccharide, z.B. Xanthum Gummi, Caprylic/Capric Triglyceride, Sodium acrylates Copolymer, Polyquaternium-32 (and) Paraffinum Liquidum (INCI), Sodium Acrylates Copolymer (and) Paraffinum Liquidum (and) PPG-1 Trideceth-6, Acrylamidopropyl Trimonium Chloride/Acrylamide Copolymer, Steareth-10 Allyl Ether Acrylates Copolymer, Polyquaternium-37 (and) Paraffinum Liquidum (and) PPG-1 Trideceth-6, Polyquaternium 37 (and) Propylene Glycole Dicaprate Dicaprylate (and) PPG-1 Trideceth-6, Polyquaternium-7, Polyquaternium-44.

Die erfindungsgemäßen Polymerisate können auch in Shampooformulierungen als Festigungs- und/oder Konditioniermittel eingesetzt werden. Als Konditioniermittel eignen sich insbesondere Polymere mit kationischer Ladung. Bevorzugte Shampooformulierungen enthalten

a) 0,05 - 10 Gew.-% des erfindungsgemäßen Polymerisates
b) 25 - 94,95 Gew.-% Wasser
c) 5 - 50 Gew.-% Tenside
c) 0 - 5 Gew.-% eines weiteren Konditioniermittels
d) 0 - 10 Gew.-% weitere kosmetische Bestandteile

In den Shampooformulierungen können alle in Shampoos üblicherweise eingesetzte anionische, neutrale, amphotere oder kationische Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Geeignet sind zum Beispiel Natriumlaurylsulfat, Ammoniumlaurylsulfat, Natriumlaurylethersulfat, Ammoniumlaurylethersulfat, Natriumlauroylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate. Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, Alkylpolyglykoside oder Sorbitanetherester geeignet.

Außerdem können die Shampooformulierungen übliche kationische Tenside enthalten, wie z.B. quaternäre Ammoniumverbindungen, beispielsweise Cetyltrimethylammoniumchlorid.

In den Shampooformulierungen können zur Erzielung bestimmter Effekte übliche Konditioniermittel in Kombination mit den erfindungsgemäßen Polymerisaten eingesetzt werden. Hierzu zählen beispielsweise kationische Polymere mit der Bezeichnung Polyquaternium nach INCI, insbesondere Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/ Dimethylaminoethylmethacrylat, quaternisiert mit Diethylsulfat (Luviguat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidcopolymere (Polyquaternium-7). Ferner können Eiweißhydrolysate verwendet werden, sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon Copolyole (CTFA) und aminofunktionelle Silikonverbindungen wie Amodimethicone (CTFA). Ferner können kationische Guarderivate wie Guarhydroxypropyltrimoniumchlorid (INCI) verwendet werden.

Ausführungsbeispiele

Nachfolgende Beispiele belegen die verbesserten Eigenschaften der erfindungsgemäßen Polymerisate im Vergleich zu Polymerisaten bei denen entweder Wasser in größeren Mengen im Reaktionsgemisch vorlag oder die Polyetherkomponente erst nach Polymerisation der einzelnen Monomere hinzugegeben wurde.

Eingesetzt wurden:

a) Vinylimidazol quaternisiert mit Dimethylsulfat, 45 %ige Lösung in Wasser (infolge "QVIXDMS")

b) Diallyldimethylammoniumchlorid, 65 %ige Lösung in Wasser (infolge "DADMAC")

c) Pluronic® PE 4300 Polyethylenglykol-Polypropylenglykol-Blockcopolymer (BASF Aktiengesellschaft)

d) Pluriol® E 600 Polyethylenglykol mit Molekulargewicht 600 (BASF Aktiengesellschaft)

e) Wako V 50 Azostarter der Fa. Wako Chemicals GmbH

Beispiel 1: Herstellung eines kationischen Polymerisates nach dem erfindungsgemäßen Verfahren.

Hergestellt wurde ein kationisches Polymerisat mit der mengenmäßigen Zusammensetzung Pluronic PE 4300: QVIxDMS:DADMAC = 92 : 7 : 1. Als Monomere (a1) wurden QVIxDMS und DADMAC im Verhältnis 7 : 1 eingesetzt. Der Wassergehalt während der Polymerisation betrug 7 Gew.-%.

| | | | Einsatzstoffkonz |
|---|---|---|---|
| Vorlage | Pluronic PE 4300 | 152,3 g | 100 % |
| | Zulauf 1 | 8,0 g | |
| Zulauf 1 | QVIxDMS 45 % | 26,2 g | 45 % |
| | DADMAC 65 % | 2,6 g | 65 % |
| Zulauf 2 | Pluriol E 600 | 30,0 g | 100 % |
| | tert.-Butylperoxiethylhexanoat | 1,35 g | 100 % |
| Zulauf 3 | VE-Wasser | 120,0 g | 100 % |

Die Reaktion wurde in einer 2 1 Glasapparatur (HWS) mit Ankerrührer und Temperaturkontrolle (innen) durchgeführt. Die Vorlage wurde mit dem flüssigen Produkt Pluronic PE 4300 und einer Teilmenge des Zulaufs 1 gefüllt und dann auf 85°C aufgeheizt. Bei 80°C wurden 8 g des Zulaufs 2 zugeben und anschließend 4 Minuten gewartet. Dann wurden die Zuläufe 1 und 2 gleichzeitig gestartet. Der Zulauf 1 wurde über 6 h, der Zulauf 2 parallel dazu auch über 6 h zugegeben. Dann wurde 2 h bei 85°C nachpolymerisiert. Anschließend wurde auf Raumtemperatur gekühlt und mit Zulauf 3 verdünnt. Beurteilung der Eigenschaften siehe unten.

Vergleichsbeispiel 1: Herstellung eines kationischen Polymerisates durch Polymerisation in wässriger Lösung

Hergestellt wurde ein kationischen Polymerisat mit der mengenmäßigen Zusammensetzung Pluronic PE 4300: QVIxDMS:DADMAC = 92 : 7 : 1. Die mengenmäßige Zusammensetzung war identisch mit der von Beispiel 1. Als Monomere (a1) wurden QVIxDMS und DADMAC im Verhältnis 7 : 1 eingesetzt. Der Wassergehalt während der Polymerisation betrug jedoch im Unterschied zu Beispiel 1 50 Gew.-%.

| | | | Einsatzstoffkonz |
|---|---|---|---|
| Vorlage | Pluronic PE 4300 | 152,3 g | 100 % |
| | Zulauf 1 | 8,0 g | |
| | VE-Wasser | 185,0 g | 100 % |
| Zulauf 1 | QVIxDMS 45 % | 26,2 g | 45 % |
| | DADMAC 65 % | 2,6 g | 65 % |
| Zulauf 2 | Pluriol E 600 | 30,0 g | 100 % |
| | tert.-Butylperoxiethylhexanoat | 1,35 g | 100 % |

Die Polymerisation wurde analog wie für Beispiel 1 beschrieben durchgeführt. Die Reaktion wurde in einer 2 1 Glasapparatur (HWS) mit Ankerrührer und Temperaturkontrolle (innen) durchgeführt. Die Vorlage wurde mit dem flüssigen Produkt Pluronic PE 4300, Wasser und einer Teilmenge des Zulaufs 1 gefüllt und dann auf 85°C aufgeheizt. Bei 80°C wurden 8 g des Zulaufs 2 zugeben und anschließend 4 Minuten gewartet. Dann wurden die Zuläufe 1 und 2 gleichzeitig gestartet. Der Zulauf 1 wurde über 6 h, der Zulauf 2 parallel dazu auch über 6 h zugegeben. Dann wurde 2 h bei 85°C nachpolymerisiert. Anschließend wurde auf Raumtemperatur gekühlt. Beurteilung der Eigenschaften siehe unten.

Vergleichsbeispiel 2: Herstellung einer Mischung aus Polyether und einem kationischen Polymer

a) Hergestellt wurde ein kationischen Polymerisat mit der mengenmäßigen Zusammensetzung QVIxDMS:DADMAC =

92:7:1. Dieses dient als Vorprodukt für die Herstellung einer Mischung mit der Polyetherkomponente. Die mengenmäßige Zusammensetzung war identisch mit der der Monomere (a1) in Beispiel 1. Als Monomere (a1) wurden QVIxDMS und DADMAC im Verhältnis 7:1 eingesetzt.

| | | | Einsatzstoffkonz |
|---|---|---|---|
| Vorlage | Zulauf 1 | 12,0 g | |
| | VE-Wasser | 230,0 g | 100 % |
| Zulauf 1 | QVIxDMS 45 % | 60,9 g | 45 % |
| | DADMAC 65 % | 6,02 g | 65 % |
| Zulauf 2 | VE Wasser | 20,0 g | 100 % |
| | Wako V50 | 0,65 g | 100 % |

Die Reaktion wurde in einer 2-1-Glasapparatur (HWS) mit Ankerrührer und Temperaturkontrolle (innen) durchgeführt. Die Vorlage wurde mit Wasser und einer Teilmenge des Zulaufs 1 gefüllt und dann auf 75°C aufgeheizt. Bei 70°C wurden 2 g des Zulaufs 2 zugeben und anschließend 3 Minuten gewartet. Dann wurden die Zuläufe 1 und 2 gleichzeitig gestartet. Der Zulauf 1 wurde über 2,5 h, der Zulauf 2 über 3 h zugegeben. Dann wurde bei 75°C 3 h nachpolymerisiert. Anschließend wurde auf Raumtemperatur gekühlt. Beurteilung der Eigenschaften des kationischen Vorduktes siehe unten.

b) Es wurde eine Mischung aus dem wässrigen, kationischen polymeren Vorprodukt (s.o.) und Pluronic PE 4300 mit der formalen Zusammensetzung wie in Beispiel 1 oder Vergleichsbeispiel 1 hergestellt. Die Zusammensetzung betrug Pluronic PE 4300: QVIxDMS:DADMAC = 92 : 7 : 1.

| | | | Einsatzstoffkonz |
|---|---|---|---|
| Vorlage | Pluronic PE 4300 | 92,0 g | 100 % |
| | kat. | 74,4 g | 10,8 % |
| | Pluriol E 600 | 18,1 g | 100 % |
| | VE-Wasser | 10,0 g | 100 % |

Die Mischung wurde in einer 2-1-Glasapparatur (HWS) mit Ankerrührer und Temperaturkontrolle (innen) durchgeführt. In die Vorlage wurde neben den Produkten Pluronic PE 4300 und Pluriol E 600 das kationische Vorprodukt aus dem vorangegangenen Versuch eingefüllt. Diese Vorlage wird intensiv bei Raumtemperatur gemischt. Beurteilung der Eigenschaften siehe unten.

Vergleichsbeispiel 3: Nachstellung von Beispiel 2 aus US 4,048,301

Beispiel 2 aus US 4,048,301 wurde wie dort beschrieben nachgestellt. Dazu wurden in einem 500 ml Rundkolben mit mechanischem Rührer, Rückflußkühler und Thermometer die nachfolgender Edukte eingeführt:

- N-Vinylpyrrolidon, frisch destilliert 54,62 g
- Dimethylaminoethylmethacrylat 9,87 g
- Dimethylaminoethylmethacrylat quaternisiert mit Dimethylsulfat 26,70 g
- Polyethyleneglycol, MW-20,000 8,81 g
- Azo-bis-isobutyronitril 0,2 g
- Ethanol, absolute 20 g

Das Reaktionsgemisch wurde unter Rühren auf 65°C erwärmt. Sobald die Mischung viskos wird, werden nochmals auf 65°C vorgewärmte 80 g abs. Ethanol zugegeben. Die Temperatur wird dann auf 76°C erhöht und dort unter Rühren für 24 h gehalten. Anschließend werden 200 g Wasser zugegeben und das Wasser-Ethanol Azeotrop abdistilliert bis sämtlicher Ethanol entfernt wurde.

Beispiel 2: Vergleich der Eigenschaften der erfindungsgemäßen Polymerisate mit den Polymerisaten der Vergleichsbeispiele 1, 2 und 3

Die Polymerisate wurden in einer Tensidlösung-Rezeptur mit nachfolgender Zusammensetzung eingesetzt:

| | |
|---|---|
| 40,0 % | Texapon NSO (Sodium Laureth Sulfat Lösung 28 %; Cognis) |
| 10,0 % | Tego-Betain L7 (Cocamidopropyl Betain Lösung 30 %; Goldschmidt) |
| 0,5 % | Polymerisat (Feststoffgehalt) |
| add 100 % | Wasser |

i) Bestimmung der Kämmbarkeit

Die folgende Arbeitsanleitung beschreibt die Vorgehensweise zur Bestimmung der Nass- und Trockenkämmbarkeit von Haaren nach der Behandlung mit Konditioniermitteln. Alle Messungen werden im Klimaraum bei 65 % relativer Feuchte und 21°C durchgeführt.

Verwendete Geräte

Nasskämmbarkeit: Frank Zug/Druck-Prüfgerät
Trockenkämmbarkeit: Diastron Kraftmesssystem
Digitalwaage (Oberschalenwaage)

Haare:

a) europäisch, gebleicht: Haartressen der Fa. Wernesgrün (Bleichung siehe unten)

b) asiatisch, unbehandelt: Haartressen der Fa. Wernesgrün mit gesplissten Spitzen

Folgende Prüfungen werden durchgeführt:

- Nasskämmbarkeit nach Shampooanwendung an europischen, gebleichten Haaren

- Trockenkämmbarkeit nach Shampooanwendung an asiatischen Haaren

Vorbehandlung/ Reinigung der Haare:

Vor der Erstbenutzung werden die asiatischen Haartressen in einem Lösungsmittelgemisch (Ethanol/Isopropanol/Aceton/Wasser 1 : 1 : 1 : 1) gereinigt bis die Haare im trockenen Zustand sauber (d.h. nicht mehr verklebt) aussehen. Anschließend werden die Haare mit Natriumlaurylethersulfat gewaschen.

Die europäischen Haare werden danach mit einer Bleichpaste (7,00 g Ammoniumcarbonat, 8,00 g Calciumcarbonat, 0,50 g Aerosil 200, 9,80 g Wasserstoffperoxid (30 %ig), 9,80 g Vollentsalztes Wasser) behandelt. Die Haartressen werden in die Bleichpaste vollständig eingetaucht, so dass eine umfangreiche Benetzung der gesamten Haaroberfläche gewährleistet ist. Anschließend werden die Tressen zwischen den Fingern abgestreift um die überschüssige Bleichpaste zu entfernen. Die Einwirkzeit, des somit verbleibenden Bleichmittels auf dem Haar, wird dem Grad der benötigten Schädigung angepasst, beträgt in der Regel 15 bis 30 Minuten, kann aber bedingt durch die Haarqualität schwanken. Danach werden die gebleichten Haartressen unter fließendem Leitungswasser gründlich (2 Minuten) gespült und mit Natriumlaurylethersulfat gewaschen. Anschließend sollten die Haare wegen der sogenannten schleichenden Bleiche kurz in einer wässrigen, sauren Lösung (z.B. Citronensäure) eingetaucht und mit Leitungswasser nachgespült werden.

Anwendungen:

In das Haar wird 1 Minute in die zu testende Tensidformulierung getaucht, 1 Minute shampooniert und anschließend 1 Minute unter fließendem Trinkwasser (handwarm) ausgespült.

I) Nasskämmbarkeit

Bestimmung Blindwert Nasskämmbarkeit: Die gewaschenen Haare werden über Nacht im Klimaraum getrocknet. Vor der Messung werden sie zweimal mit Texapon NSO insgesamt, 1 Minute shampooniert und 1 Minute ausgespült, damit sie definiert nass, d.h. gequollen sind. Vor Beginn der Messung wird die Tresse so vorgekämmt, bis keine Verhakungen der Haare mehr vorhanden sind und somit bei wiederholtem Messkämmen eine konstante Kraftaufwendung erforderlich ist. Anschließend wird die Tresse an der Halterung fixiert und mit der feinzinkigen Seite des Kammes in die feinzinkige Seite des Prüfkammes eingekämmt. Das Einlegen der Haare in den Prüfkamm hat bei jeder Messung gleichmäßig und spannungsfrei zu erfolgen. Die Messung wird gestartet und mittels Software (EGRA-NUDO-Programm, Fa. Frank) ausgewertet. Die Einzelmessung wird 5 bis 10 mal wiederholt. Der errechnete Mittelwert wird notiert.

Bestimmung Messwert Nasskämmbarkeit: Nach der Bestimmung des Blindwertes werden die Haare je nach gewünschter Anwendung behandelt. Die Messung der Kämmkraft erfolgt analog der Blindwertbestimmung.
Auswertung:

$$\text{Kämmkraftabnahme nass [\%]} = 100 - (\text{Messwert} * 100/\text{Blindwert})$$

II) Trockenkämmbarkeit

Bestimmung Blindwert Trockenkämmbarkeit: Die gewaschenen Haare werden über Nacht im Klimaraum getrocknet. Vor Beginn der Messung wird die Tresse so vorgekämmt, bis keine Verhakungen der Haare mehr vorhanden sind und somit bei wiederholtem Messkämmen eine konstante Kraftaufwendung erforderlich ist. Anschließend wird die Tresse an der Halterung fixiert und in die feinzinkige Seite des Prüfkamms eingekämmt. Das Einlegen der Haare in den Prüfkamm hat bei jeder Messung gleichmäßig und spannungsfrei zu erfolgen. Die Messung wird gestartet und mittels Software (mtt-win, Fa. DIASTRON) ausgewertet. Die Einzelmessung wird 5- bis 10 mal wiederholt. Der errechnete Mittelwert wird zusammen mit der Standardabweichung notiert.

Bestimmung Messwert Trockenkämmbarkeit: Nach der Bestimmung des Blindwertes werden die Haare je nach gewünschter Anwendung behandelt und über Nacht getrocknet. Die Messung der Kämmkraft erfolgt analog der Blindwertbestimmung. Auswertung:

$$\text{Kämmkraftabnahme nass [\%]} = 100 - (\text{Messwert} * 100/\text{Blindwert})$$

Das erfindungsgemäße Polymerisat liefert, die Trockenkämmbarkeit, insbesondere aber die Nasskämmbarkeit betreffend, hervorragende Resultate. Ein weiterer Vorzug ist, dass mit dem erfindungsgemäßen Polymerisat auch klare (Wasch-) Formulierungen möglich sind. Das in US 4,048,301 beschriebene Polymer (Vergleich 3) ist zwar den Vergleichsbeispielen 1 und 2 überlegen, ist jedoch dem erfindungsgemäßen Polymer deutlich unterlegen.

| | Beispiel 1 | Vergleich 1 | Vergleich 2a | Vergleich 2b | Vergleich 3 |
|---|---|---|---|---|---|
| Festgehalt (Gew.-%) | 60,2 | 49,8 | 10,8 | 60,8 | 37,2 |
| Kämmkraftabnahme Nass (%) (europ. Haar) | 44 | 23 | 15 | 18 | 28 |
| Kämmkraftabnahme Trocken (%) (asiat. Haar) | 86 | 77 | ND | ND | 79 |
| Tensidlösung 0,5 % Wirkstoff | klar | klar | leicht trüb | klar | klar |
| K-Wert 1 % in Ethanol | 15,1 | | | | |
| ND: Nicht bestimmt, da hämmkraftabnahme nass ungenügend (<20 %) | | | | | |

## Patentansprüche

**1.** Verwendung von kationischen Polymerisaten, erhältlich durch Polymerisation von 3 bis 30 Gew.-% mindestens

eines quaternären Stickstoff enthaltenden radikalisch polymerisierbaren Monomers (a1) und/oder eines direkten Vorproduktes (a2) desselben
in Gegenwart von 70 bis 97 Gew.-% mindestens einer polyetherhaltigen Verbindung (b), ausgewählt aus der Gruppe bestehend aus
Verbindungen der allgemeinen Formel I

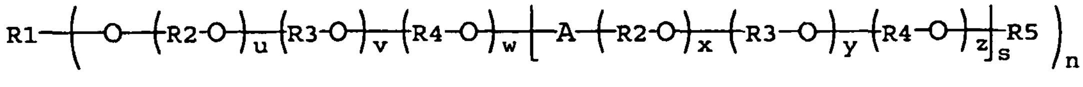

(I)

in der die Variablen unabhängig voneinander folgende Bedeutung haben:

$R^1$ Wasserstoff, $C_1$-$C_{24}$-Alkyl, $R^6$-C(=O)-, $R^6$-NH-C(=O)-, Polyalkoholrest;
$R^5$ Wasserstoff, $C_1$-$C_{24}$-Alkyl, $R^6$-C(=O)-, $R^6$-NH-C(=O)-;
$R^2$ bis $R^4$ -$(CH_2)_2$-, -$(CH_2)_3$- -$(CH_2)_4$-, -$CH_2$-CH($R^6$)-, -$CH_2$-CHOR$^7$-$CH_2$-;
$R^6$ $C_1$-$C_{24}$-Alkyl;
$R^7$ Wasserstoff, $C_1$-$C_{24}$-Alkyl, $R^6$-C(=O)-, $R^6$-NH-C(=O)-;
A -C(=O)-O, -C(=O)-B-C(=O)-O, -$CH_2$-CH(-OH)-B-CH(-OH)-$CH_2$-O, -C(=O)-NH-B-NH-C(=O)-O;

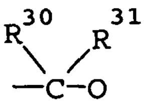 ;

B -$(CH_2)_t$-, Arylen, ggf. substituiert;
$R^{30}$, $R^{31}$ Wasserstoff, $C_1$-$C_{24}$-Alkyl, $C_1$-$C_{24}$-Hydroxyalkyl, Benzyl oder Phenyl;
n 1 wenn $R^1$ kein Polyakoholrest ist oder
n 1 bis 1000 wenn $R^1$ ein Polyakoholrest ist
s 0 bis 1000;
t 1 bis 12;
u 1 bis 5000;
v 0 bis 5000;
w 0 bis 5000;
x 0 bis 5000;
y 0 bis 5000;
z 0 bis 5000,

Polyetherhaltigen Silikonderivaten und
Umsetzungsprodukten von Polyethyleniminen mit Alkylenoxiden,
gegebenenfalls 0 bis 15 Gew.-% eines oder mehrerer weiterer radikalisch polymerisierbarer Monomere (c) mit einer Wasserlöslichkeit oberhalb 60 g/l bei 25°C und
gegebenenfalls 0 bis 15 Gew.-% eines oder mehrerer weiterer radikalisch polymerisierbarer Monomere (d) mit einer Wasserlöslichkeit unterhalb 60 g/l bei 25°C
wobei der Wassergehalt im Reaktionsgemisch während der Polymerisation weniger als 20 Gew.-% beträgt, und
wobei bei Verwendung eines Vorproduktes (a2) dieses im Anschluss an oder während der Polymerisation zumindest teilweise in eine Verbindung mit quaternärem Stickstoff (a2') umgesetzt wird, und
wobei das molare Verhältnis der Summe der Monomeren (a1), (a2') und (c) zu der Summe der Monomeren (d) mindestens 2 zu 1 beträgt, und
wobei sich die Gew.-% der einzelnen Komponenten a1 und/oder a2, b und gegebenenfalls c und d auf jeweils 100 Gew.-% addieren,
in kosmetischen Zubereitungen.

2. Verwendung nach Anspruch 1, wobei das Monomer (a1) und/oder die Verbindung (a2') ausgewählt ist aus der

Gruppe bestehend aus quaternären Vinylaminen, N,N,N-Trialkylaminoalkylacrylate und -methacrylate, N,N,N-Trialkylaminoalkylacrylamide und -methacrylamide, 3-Alkyl-1-vinylimidazolen, 3-Aryl-1-vinylimidazolen, quaternären Vinylpyridinen und quaternären Diallylaminen, sowie der Salzen.

**3.** Verwendung nach Anspruch 1 oder 2, wobei das Monomer (a1) und/oder die Verbindung (a2') ausgewählt ist aus der Gruppe bestehend aus

a) quaternäre Vinylaminen der allgemeinen Formeln (IIIa) oder (IIIb)

$$W\text{-}(CH_2)_n\text{-}CR^{15}\text{=}CHR^{14} \qquad \text{(IIIa)}$$

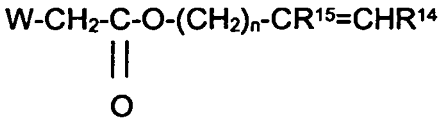 (IIIb)

wobei
$R^{14}$ und $R^{15}$ unabhängig voneinander ausgewählt sind aus der Gruppe bestehend aus Wasserstoff, $C_1$-$C_8$ linear- oder verzweigtkettige Alkyl, Methoxy, Ethoxy, 2-Hydroxyethoxy, 2-Methoxyethoxy und 2-Ethoxyethyl, und n ist 0, 1 oder 2, und
W ist $-N^+(R^{16})_3/\ X^-$ oder

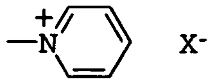

wobei die Reste $R^{16}$ identisch oder verschieden ausgewählt werden können aus der Gruppe bestehend aus $C_1$-$C_{40}$ linear- oder verzweigtkettige Alkylreste, Formyl, $C_1$-$C_{10}$ linear- oder verzweigtkettige Acyl, N,N-Dimethylaminoethyl, 2-Hydroxyethyl, 2-Methoxyethyl, 2-Ethoxyethyl, Hydroxypropyl, Methoxypropyl, Ethoxypropyl oder Benzyl, und wobei $X^-$ ein Anion ist,
b) N,N,N-Trialkylaminoalkylacrylaten oder-methacrylaten, N,N,N-Trialkylaminoalkylacrylamiden oder -methacrylamiden der allgemeine Formel (IV)

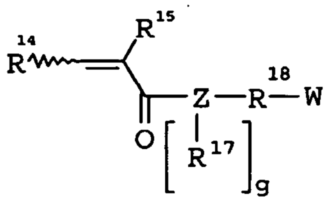 (IV)

wobei $R^{14}$. $R^{15}$ und W die gleiche Bedeutung wie in der allgemeinen Formel IIIa und IIIb in Anspruch 3 a) haben, und

$R^{17}$ = Wasserstoff oder Methyl,
$R^{18}$ = Alkylen oder Hydroxyalkylen mit 1 bis 24 C-Atomen,
Z = Stickstoff für g = 1 oder Sauerstoff für g = 0,

c) quaternären N-Vinylimidazolen der allgemeinen Formel (V)

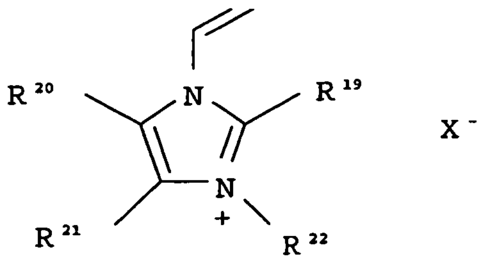

(V)

wobei
$R^{19}$ bis $R^{21}$ unabhängig voneinander für Wasserstoff, $C_1$-$C_4$-Alkyl, $C_1$-$C_4$-Hydroxyalkyl oder Phenyl; und
$R^{22}$ für $C_1$-$C_4$-Alkyl, $C_1$-$C_4$-Hydroxyalkyl oder Phenyl; und
und $X^-$ für ein Anion steht,
d) quaternären Vinylpyridinen der allgemeinen Formel (VI)

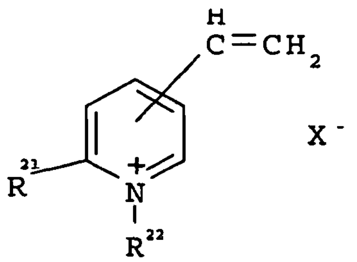

(VI)

wobei $R^{21}$, $R^{22}$ und $X^-$ die gleiche Bedeutung wie in der allgemeinen Formel (V) in Anspruch 3 c) haben,
e) quaternären Diallylaminen der allgemeinen Formel (VII)

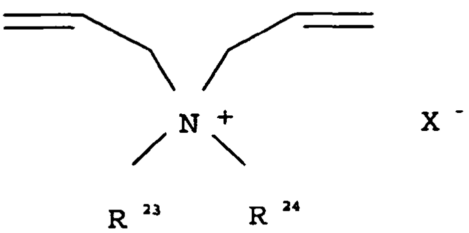

(VII)

wobei $R^{23}$ und $R^{24}$ jeweils und unabhängig voneinander $C_1$- bis $C_{24}$-Alkyl sein können und $X^-$ für ein Anion steht.

4. Verwendung nach einem der Ansprüche 1 bis 3, wobei das Monomere (a1) und/oder die Verbindung (a2') ausgewählt ist aus N,N,N-Trimethylaminomethyl(meth)acrylat, N,N,N-Triethylaminomethyl(meth)acrylat, N,N,N-Trimethylaminoethyl(meth)acrylat, N,N,N-Triethylaminoethyl-(meth)acrylat, N,N,N-Trimethylaminobutyl(meth)acrylat, N,N,N-Triethylaminobutyl(meth)acrylat, N,N,N-Trimethylaminohexyl(meth)acrylat, N,N,N-Trimethylaminooctyl(meth)acrylat, N,N,N-Trimethylaminododecyl(meth)acrylat, N-[3-(Trimethylamino)propyl]methacrylamid und N-[3-(Trimethylamino)propyl]acrylamid, N-[3-(Dimethylamino)butyl]-methacrylamid, N-[8-(Trimethylamino)octyl]methacrylamid, N-[12-(Trimethylamino)dodecyl]methacrylamid, N-[3-(Triethylamino)propyl]methacrylamid und N-[3-(Triethylamino)propyl]-acrylamid, (Meth)acryloyloxyhydroxypropyltrimethylamin, (Meth)acryloyloxyhydroxypropyltriethylamin, 3-Methyl-1-vinylimidazol und N,N-Dimethyl-N,N-diallylamin.

5. Verwendung nach einem der Ansprüche 1 bis 4, wobei die Polymerisate ausgehend von Mischungen aus 3-Methyl-1-vinylimidazoliummethylsulfat und N,N-Dimethyl-N,N-diallylammoniumchlorid als Komponente (a1) erhältlich sind.

6. Verwendung nach einem der Ansprüche 1 bis 5, wobei das Monomer (a2) ausgewählt ist aus radikalisch polymerisierbaren ungesättigten primären, sekundären und tertiären Aminen, ungesättigten Säuren und ungesättigten

Halogeniden.

7. Verwendung nach einem der Ansprüche 1 bis 6, wobei das Monomer (a2) ein Amin ist ausgewählt aus der Gruppe von Verbindungen bestehend aus

a) Aminoalkylacrylaten, Aminoalkylmethacrylaten, Aminoalkylacrylamiden und Aminoalkylmethacrylamiden der allgemeinen Formel (VIII)

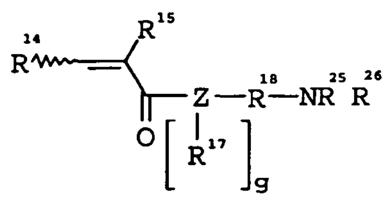

(VIII)

wobei für $R^{14}$ bis $R^{18}$ die für Formel (IV) in Anspruch 3 b) gegebenen Definitionen gelten und $R^{25}$ bzw. $R^{26}$ jeweils und unabhängig voneinander ausgewählt sind aus der Gruppe bestehend aus Wasserstoff, $C_1$-$C_{40}$ linear- oder verzweigtkettige Alkyl, Formyl, $C_1$-$C_{10}$ linear- oder verzweigtkettige Acyl, N,N-Dimethylaminoethyl, 2-Hydroxyethyl, 2-Methoxyethyl, 2-Ethoxyethyl, Hydroxypropyl, Methoxypropyl, Ethoxypropyl oder Benzyl,

b) N-Vinylimidazolen der allgemeinen Formel IX, wobei für $R^{19}$ bis $R^{21}$ unabhängig voneinander die für Formel (V) in Anspruch 3 c) gegebenen Definitionen gelten,

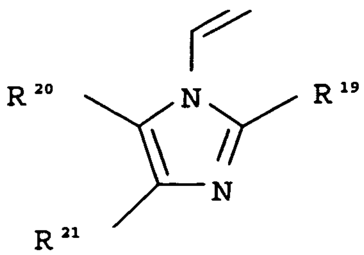

(IX)

c) Diallylaminen der allgemeinen Formel (X)

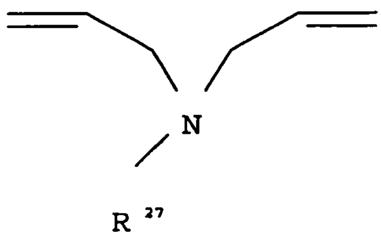

(X)

mit $R^{27}$= Wasserstoff oder $C_1$- bis $C_{24}$-Alkyl,

d) 1,3-Divinylimidazolid-2-on oder N-Disubstituierten Vinylaminen der allgemeinen Formel (XI):

$$(R^{28})_2N\text{-}(CH_2)_n\text{-}CR^{15}\text{=}CHR^{14} \qquad \text{(XI)}$$

wobei $R^{14}$, $R^{15}$ und n die gleiche Bedeutung wie in den Formeln (IIIa) und (1-IIb) haben, und die Reste $R^{28}$ ausgewählt seien können aus der Gruppe bestehend aus Wasserstoff $C_1$-$C_{40}$ linear- oder verzweigtkettige Alkylreste, Formyl, $C_1$-$C_{10}$ linear- oder verzweigtkettige Acyl, N,N-Dimethylaminoethyl, 2-Hydroxyethyl, 2-Methoxyethyl, 2-Ethoxyethyl, Hydroxypropyl, Methoxypropyl, Ethoxypropyl oder Benzyl, wobei gilt, wenn n = 0, dass nicht beide Reste $R^{28}$ gleichzeitig Wasserstoff sind.

**8.** Verwendung nach einem der Ansprüche 1 bis 7, wobei das Monomer (a2) ausgewählt ist aus N,N-Dimethylaminoethylmethacrylat, N-[3-(dimethylamino)propyl]methacrylamid, N-Methylaminoethylmethacrylat, N-[3-(Methylamino)propyl]methacrylamid, Aminoethylmethacrylat und N-[3-aminopropyl]methacrylamid, N-Vinylimidazol, 1-Vinyl-2-methylvinylimidazol und N,N-Diallylamin.

**9.** Verwendung nach einem der Ansprüche 6 bis 8, wobei die Quaternisierung mit einem Alkylhalogenid mit 1 bis 24 C-Atomen, einem Dialkylsulfat mit 1 bis 24 C-Atomen, einem Alkylenoxid oder einem Epichlorhydrin erfolgt.

**10.** Verwendung nach Anspruch 6, wobei das Monomer (a2) ein ungesättigte Halogenid ist ausgewählt aus den Halogenalkylacrylate oder Halogenalkylmethacrylate.

**11.** Verwendung nach Anspruch 10, wobei die Quaternisierung mit einem Trialkylamin erfolgt.

**12.** Verwendung nach einem der Ansprüche 1 bis 11, wobei das Monomer (c) ausgewählt ist aus der Gruppe bestehend aus N-Vinyllactamen, N-Vinylcarbonsäureamiden, Hydroxyalkylacrylaten, ethylenisch ungesättigte Amiden, Vinylimidazolen, ungesättigte Säuren und ungesättigten Amine.

**13.** Verwendung nach einem der Ansprüche 1 bis 12, wobei das Monomer (c) ausgewählt ist aus der Gruppe bestehend aus N-Vinylpyrrolidon, N-Vinylpiperidon, N-Vinylcaprolactam, N-Vinylformamid, N-EthyhN-vinylacetamid oder N-Methyl-N-vinylacetamid, 2-Hydroxyethylacrylat, 2-Hydroxyethylmethacrylat, 2-Hydroxypropylmethacrylat, Butandiolmonoacrylat, Acrylamid, Methacrylamid, N-Vinylimidazol, Acrylsäure, Maleinsäure, Methacrylsäure, 2-Acrylamido-2-methylpropansulfonsäure, Dimethylaminoethylacrylat und Dimethylaminomethacrylat.

**14.** Verwendung nach einem der Ansprüche 1 bis 13, wobei das Monomer (d) ausgewählt ist aus der Gruppe bestehend aus $C_1$-$C_{10}$-Alkylester monoethylenisch ungesättigter $C_3$-$C_6$-Carbonsäuren, Di-$C_1$-$C_{10}$-alkylester ethylenisch ungesättigter Dicarbonsäuren, Kohlenwasserstoffe mit mindestens einer radikalisch polymerisierbaren Kohlenstoff-Kohlenstoff Doppelbindung, Vinyl-, Vinyliden- oder Allylhalogenide, Vinyl-, Allyl- und Methallylester von $C_1$-$C_{40}$ linearen, $C_3$-$C_{40}$ verzweigtkettigen oder $C_3$-$C_{40}$ carbocyclische Carbonsäuren aliphatischer, gesättigter und ungesättigter Natur, Vinyl-, Allyl- und Methallylether linearer oder verzweigter, aliphatischer Alkohole mit 2 bis 20 C-Atomen.

**15.** Verwendung nach einem der Ansprüche 1 bis 14, wobei das Monomer (d) ausgewählt ist aus der Gruppe bestehend aus Methylacrylat, Ethylacrylat, Propylacrylat, n-Butylacrylat, iso-Butylacrylat, t-Butylacrylat, 2-Ethylhexylacrylat, Decylacrylat, Methylmethacrylat, Ethylmethacrylat, Propylmethacrylat, n-Butylmethacrylat, iso-Butylmethacrylat, t-Butylmethacrylat, 2-Ethylhexylmethacrylat, Decylmethacrylat, Methylethacrylat, Ethylethacrylat, n-Butylethacrylat, iso-Butylethacrylat, t-Butyl-ethacrylat, 2-Ethylhexylethacrylat, Decylethacrylat, Stearylacrylat, Stearyl(meth)acrylat, bevorzugt Styrol, alpha-Methylstyrol, tert.-Butylstyrol, Butadien, Isopren, Cyclohexadien, Ethylen, Propylen, 1-Buten, 2-Buten, Isobutylen, Vinyltoluol, Vinylchlorid, Vinylidenchlorid, Allylchlorid, Vinylacetat, Vinylpropionat, Vinylbutyrat, Vinylvalerat, Vinylhexanoat, Vinyl-2-ethylhexanoat, Vinyldecanoat, Vinyllaurat, Vinylstearat, Vinylmethylether, Vinylethylether, Vinyldodecylether, Vinylhexadecylether, Vinylstearylether, Acrylamidoglycolsäure, Fumarsäure und Crotonsäure.

**16.** Verwendung nach einem der Ansprüche 1 bis 15, wobei die polyetherhaltige Verbindung (b) beschrieben durch die allgemeine Formel I ein mittleres Molekulargewicht von 500 bis 50000 (nach dem Zahlenmittel) hat und die Variablen unabhängig voneinander folgende Bedeutung haben:

$R^1$ Wasserstoff, $C_1$-$C_6$-Alkyl, $R^6$-C(=O)-, $R^6$-NH-C(=O)-;
$R^5$ Wasserstoff, $C_1$-$C_6$-Alkyl, $R^6$-C(=O)-, $R^6$-NH-C(=O)-;
$R^2$ bis $R^4$ $-(CH_2)_2-$, $-(CH_2)_3-$, $-(CH_2)_4-$, $-CH_2-CH(R^6)-$, $-CH_2-CHOR^7-CH_2-$;
$R^6$ $C_1$-$C_6$-Alkyl;
$R^7$ Wasserstoff, $C_1$-$C_6$-Alkyl, $R^6$-C(=O)-, $R^6$-NH-C(=O)-;
n 1;
s 0;
u 5 bis 500;
v 0 bis 500;
w 0 bis 500.

**17.** Verwendung nach einem der Ansprüche 1 bis 16, wobei die polyetherhaltige Verbindung ein Polymerisat, Copolymerisat oder Blockcopolymerisat mindestens einer Verbindung ausgewählt aus Ethylenoxid und Propylenoxid ist.

**18.** Verwendung von Polymerisaten nach einem der Ansprüche 1 bis 17, wobei die Mengenverhältnisse

a1) 3 - 30 Gew.-%
b) 70 - 97 Gew.-%
c) 0 - 15 Gew.-%
d) 0 -15 Gew.-%

betragen und sich die Gew.-% der einzelnen Komponenten a1, b und gegebenenfalls c und d auf jeweils 100 Gew.-% addieren.

**19.** Verwendung von Polymerisaten nach einem der Ansprüche 1 bis 18, wobei die Mengenverhältnisse

a1) 4 -12 Gew.-%
b) 88 - 96 Gew.-%
c) 0 Gew.-%
d) 0 Gew.-%

betragen und sich die Gew.-% der einzelnen Komponenten a1 und b auf jeweils 100 Gew.-% addieren.

**20.** Kationisches Polymerisat, erhältlich durch Polymerisation von
3 bis 30 Gew.-% mindestens eines kationischen, quarternären, radikalisch polymerisierbaren Monomers (a1)
in Gegenwart von 70 bis 97 Gew.-% mindestens einer polyetherhaltigen Verbindungen (b) ausgewählt aus der Gruppe bestehend aus
Verbindungen der allgemeinen Formel I

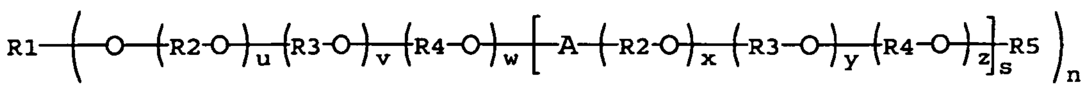

(I)

in der die Variablen unabhängig voneinander folgende Bedeutung haben:

$R^1$ Wasserstoff, $C_1$-$C_{24}$-Alkyl, $R^6$-C(=O)-, $R^6$-NH-C(=O)-, Polyalkoholrest;
$R^5$ Wasserstoff, $C_1$-$C_{24}$-Alkyl, $R^6$-C(=O)-, $R^6$-NH-C(=O)-;
$R^2$ bis $R^4$ -$(CH_2)_2$-, -$(CH_2)_3$-, -$(CH_2)_4$-, -$CH_2$-CH($R^6$)-, -$CH_2$-CHO$R^7$-$CH_2$-;
$R^6$ $C_1$-$C_{24}$-Alkyl;
$R^7$ Wasserstoff, $C_1$-$C_{24}$-Alkyl, $R^6$-C(=O)-, $R^6$-NH-C(=O)-;
A -C(=O)-O, -C(=O)-B-C(=O)-O, -$CH_2$-CH(-OH)-B-CH(-OH)-$CH_2$-O, -C(=O)-NH-B-NH-C(=O)-O;

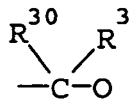 ;

B -$(CH_2)_t$-, Arylen, ggf. substituiert;
$R^{30}$, $R^{31}$ Wasserstoff, $C_1$-$C_{24}$-Alkyl, $C_1$-$C_{24}$-Hydroxyalkyl, Benzyl oder Phenyl;
n 1 wenn $R^1$ kein Polyakoholrest ist oder
n 1 bis 1000 wenn $R^1$ ein Polyakoholrest ist
s 0 bis 1000;
t 1 bis 12;
u 1 bis 5000;
v 0 bis 5000;
w 0 bis 5000;
x 0 bis 5000;
y 0 bis 5000;

z 0 bis 5000.

Polyetherhaltigen Silikonderivaten und
Umsetzungsprodukten von Polyethyleniminen mit Alkylenoxiden,
gegebenenfalls 0 bis 15 Gew.-% eines oder mehrerer weiterer radikalisch polymerisierbarer Monomere (c) mit einer Wasserlöslichkeit oberhalb 60 g/l bei 25°C und
gegebenenfalls 0 bis 15 Gew.-% eines oder mehrerer weiterer radikalisch polymerisierbarer Monomere (d) mit einer Wasserlöslichkeit unterhalb 60 g/l bei 25°C,
wobei das molare Verhältnis der Summe der Monomeren (a1) und (c) zu der Summe der Monomeren (d) mindestens 2 zu 1 beträgt,
wobei der Wassergehalt im Reaktionsgemisch während der Polymerisation weniger als 20 Gew.-% beträgt, und wobei sich die Gew.-% der einzelnen Komponenten a1, b und ggf. c und d auf jeweils 100 Gew.-% ergänzen.

**21.** Kationisches Polymerisat nach Anspruch 20, wobei

i) die Monomere (a1) wie in einem der Ansprüche 2 bis 5
ii) die polyetherhaltige Verbindung (b) wie in einem der Ansprüche 1, 16, 17,
iii) die Monomere (c) wie in einem der Ansprüche 12 oder 13, und/oder
iv) die Monomere (d) wie in einem der Ansprüche 14 oder 15

definiert sind.

**22.** Kationisches Polymerisat nach einem der Ansprüche 20 oder 21, wobei die Zusammensetzung des Polymerisates wie in einem der Ansprüche 18 oder 19 definiert ist.

**23.** Verfahren zur Herstellung von kationischen Polymerisaten, nach einem der Ansprüche 20 bis 22, **dadurch gekennzeichnet, dass**
3 bis 30 Gew.-% mindestens eines kationischen, quarternären radikalisch polymerisierbaren Monomers (a1)
in Gegenwart von 70 bis 97 Gew.-% mindestens einer polyetherhaltigen Verbindungen (b) und
gegebenenfalls 0 bis 15 Gew.-% eines oder mehrerer weiterer radikalisch polymerisierbarer Monomere (c) mit einer Wasserlöslichkeit oberhalb 60 g/l bei 25°C und
gegebenenfalls 0 bis 15 Gew.-% eines oder mehrerer weiterer radikalisch polymerisierbarer Monomere (d) mit einer Wasserlöslichkeit unterhalb 60 g/l bei 25°C,
polymerisiert wird, wobei das molare Verhältnis der Summe der Monomeren (a1) und (c) zu der Summe der Monomeren (d) mindestens 2 zu 1 beträgt,
wobei der Wassergehalt im Reaktionsgemisch während der Polymerisation weniger als 20 Gew.-% beträgt, und wobei sich die Gew.-% der einzelnen Komponenten a1, b und ggf. c und d auf jeweils 100 Gew.-% ergänzen.

**24.** Haarkosmetische Formulierung, die wie folgt zusammengesetzt sind:

| | | |
|---|---|---|
| a) | 0,05 - 20 Gew.-% | eines kationischen Polymerisates entsprechend einem der Ansprüche 1 bis 22 |
| b) | 20 - 99,95 Gew.-% | Wasser und/oder Alkohol |
| c) | 0 - 79,05 Gew.-% | weitere Bestandteile |

**25.** Haarkosmetische Formulierung, die wie folgt zusammengesetzt sind:

| | | | |
|---|---|---|---|
| a) | 0,1 -10 | Gew.-% | eines kationischen Polymerisates gemäß einem der Ansprüche 1 bis 22 |
| b) | 20 - 99,9 | Gew.-% | Wasser und/oder Alkohol |
| c) | 0 - 70 | Gew.-% | eines Treibmittel |
| d) | 0 - 20 | Gew.-% | weiterer Bestandteile |

**26.** Haarkosmetische Formulierung, die wie folgt zusammengesetzt sind:

| | | | |
|---|---|---|---|
| a) | 0,1-10 | Gew.-% | eines kationischen Polymerisates gemäß einem der Ansprüche 1 bis 22 |
| b) | 55 - 94,8 | Gew.-% | Wasser und/oder Alkohol |
| c) | 5 - 20 | Gew.-% | eines Treibmittel |
| d) | 0,1 - 5 | Gew.-% | eines Emulgators |
| e) | 0 - 10 | Gew.-% | weiterer Bestandteile |

**27.** Haarkosmetische Formulierung, die wie folgt zusammengesetzt ist:

| | | | |
|---|---|---|---|
| a) | 0,1-10 | Gew.-% | eines kationischen Polymerisates gemäß einem der Ansprüche 1 bis 22 |
| b) | 60 - 99,85 | Gew.-% | Wasser und/oder Alkohol |
| c) | 0,05 -10 | Gew.-% | eines Gelbildners |
| d) | 0 - 20 | Gew.-% | weitere Bestandteile |

**28.** Haarkosmetische Formulierung, die wie folgt zusammengesetzt ist:

| | | | |
|---|---|---|---|
| a) | 0,05-10 | Gew.-% | eines kationischen Polymerisates gemäß einem der Ansprüche 1 bis 22 |
| b) | 25 - 94,95 | Gew.-% | Wasser |
| c) | 5 - 50 | Gew.-% | Tenside |
| d) | 0 - 5 | Gew.-% | eines weiteren Konditioniermittels |
| e) | 0 -10 | Gew.-% | weiterer kosmetische Bestandteile |

## Claims

**1.** The use of cationic polymers obtainable by polymerization of
from 3 to 30% by weight of at least one quaternary nitrogen-containing free-radically polymerizable monomer (a1) and/or a direct preproduct (a2) thereof
in the presence of from 70 to 97% by weight of at least one polyether-containing compound (b) selected from the group consisting of
compounds of the formula I

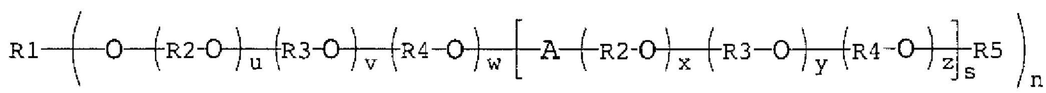

(I)

in which the variables, independently of one another, have the following meanings:

$R^1$ is hydrogen, $C_1$-$C_{24}$-alkyl, $R^6$-C(=O)-, $R^6$-NH-C(=O)-, polyalcohol radical;
$R^5$ is hydrogen, $C_1$-$C_{24}$-alkyl, $R^6$-C(=O)-, $R^6$-NH-C(=O)-;
$R^2$ to $R^4$ are $-(CH_2)_2-$, $-(CH_2)_3-$, $-(CH_2)_4-$, $-CH_2-CH(R^6)-$, $-CH_2-CHOR^7-CH_2-$;
$R^6$ is $C_1$-$C_{24}$-alkyl;
$R^7$ is hydrogen, $C_1$-$C_{24}$-alkyl, $R^6$-C(=O)-, $R^6$-NH-C(=O)-;
A is -C(=O)-O, -C(=O)-B-C(=O)-O, $-CH_2-CH(-OH)-B-CH(-OH)-CH_2-O$, -C(=O)-NH-B-NH-C(=O)-O;

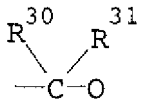 ;

B is $-(CH_2)_t-$, arylene, optionally substituted;
$R^{30}$, $R^{31}$ are hydrogen, $C_1$-$C_{24}$-alkyl, $C_1$-$C_{24}$-hydroxyalkyl, benzyl or phenyl;
n is 1 when $R^1$ is not a polyalcohol radical or
n is 1 to 1 000 when $R^1$ is a polyalcohol radical
s is 0 to 1 000;
t is 1 to 12;
u is 1 to 5 000;
v is 0 to 5 000;
w is 0 to 5 000;
x is 0 to 5 000;
y is 0 to 5 000;
z is 0 to 5 000,

polyether-containing silicone derivatives and
reaction products of polyethyleneimines with alkylene oxides,
if appropriate from 0 to 15% by weight of one or more further free-radically polymerizable monomers (c) with a solubility in water above 60 g/l at 25°C and
if appropriate from 0 to 15% by weight of one or more further free-radically polymerizable monomers (d) with a solubility in water of less than 60 g/l at 25°C
where the water content in the reaction mixture during the polymerization is less than 20% by weight, and
where, in the case of the use of a preproduct (a2), this is converted at least partially into a compound containing quaternary nitrogen (a2') subsequently to or during the polymerization, and
where the molar ratio of the sum of the monomers (a1), (a2') and (c) to the sum of the monomers (d) is at least 2 to 1, and
where the percentages by weight of the individual components a1 and/or a2, b and if appropriate c and d add up in each case to 100% by weight
in cosmetic preparations.

2. The use according to claim 1, where the monomer (a1) and/or the compound (a2') is selected from the group consisting of quaternary vinylamines, N,N,N-trialkylaminoalkyl acrylates and methacrylates, N,N,N-trialkylaminoalkylacrylamides and -methacrylamides, 3-alkyl-1-vinylimidazoles, 3-aryl-1-vinylimidazoles, quaternary vinylpyridines and quaternary diallylamines, and the salts.

3. The use according to claim 1 or 2, where the monomer (a1) and/or the compound (a2') is selected from the group consisting of

a) quaternary vinylamines of the formulae (IIIa) or (IIIb)

$$W-(CH_2)_n-CR^{15}=CHR^{14} \qquad \text{(IIIa)}$$

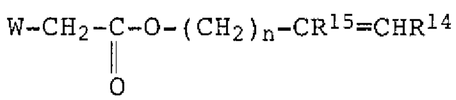 (IIIb)

where
$R^{14}$ and $R^{15}$, independently of one another, are selected from the group consisting of hydrogen, $C_1$-$C_8$ linear- or branched-chain alkyl, methoxy, ethoxy, 2-hydroxyethoxy, 2-methoxyethoxy and 2-ethoxyethyl, and
n is 0, 1 or 2, and
W is $-N^+(R^{16})_3$ / $X^-$ or

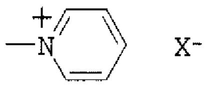

where the radicals $R^{16}$ may be selected to be identical or different from the group consisting of $C_1$-$C_{40}$ linear- or branched-chain alkyl radicals, formyl, $C_1$-$C_{10}$ linear- or branched-chain acyl, N,N-dimethylaminoethyl, 2-hydroxyethyl, 2-methoxyethyl, 2-ethoxyethyl, hydroxypropyl, methoxypropyl, ethoxypropyl or benzyl, and where $X^-$ is an anion,

b) N,N,N-trialkylaminoalkyl acrylates or methacrylates, N,N,N-trialkylaminoalkylacrylamides or -methacrylamides of the formula (IV)

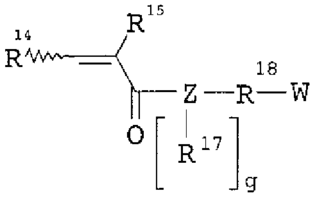

(IV)

where $R^{14}$, $R^{15}$ and W have the same meanings as in the formulae IIIa and IIIb in claim 3 a), and

$R^{17}$ = hydrogen or methyl,
$R^{18}$ = alkylene or hydroxyalkylene having 1 to 24 carbon atoms,
Z = nitrogen when g = 1 or oxygen when g = 0,

c) quaternary N-vinylimidazoles of the formula (V)

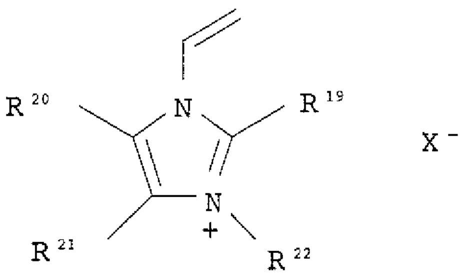

(V)

where
$R^{19}$ to $R^{21}$, independently of one another, are hydrogen, $C_1$-$C_4$-alkyl, $C_1$-$C_4$-hydroxyalkyl or phenyl; and
$R^{22}$ is $C_1$-$C_4$-alkyl, $C_1$-$C_4$-hydroxyalkyl or phenyl; and
$X^-$ is an anion,

d) quaternary vinylpyridines of the formula (VI)

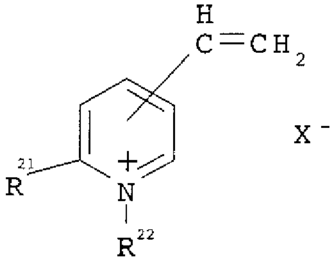

(VI)

where $R^{21}$, $R^{22}$ and $X^-$ have the same meanings as in the formula (V) in claim 3 c),
e) quaternary diallylamines of the formula (VII)

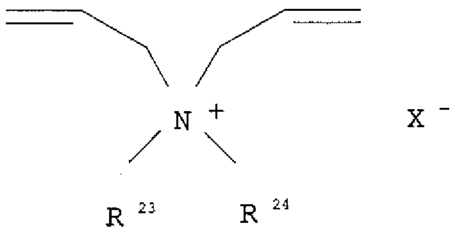

(VII)

where $R^{23}$ and $R^{24}$ in each case and independently of one another may be $C_1$- to $C_{24}$-alkyl, and $X^-$ is an anion.

**4.** The use according to any of claims 1 to 3, where the monomer (a1) and/or the compound (a2') is selected from N,N,N-trimethylaminomethyl (meth)acrylate, N,N,N-triethylaminomethyl (meth)acrylate, N,N,N-trimethylaminoethyl (meth)acrylate, N,N,N-triethylaminoethyl (meth)acrylate, N,N,N-trimethylaminobutyl (meth)acrylate, N,N,N-triethylaminobutyl (meth)acrylate, N,N,N-trimethylaminohexyl (meth)acrylate, N,N,N-trimethylaminooctyl (meth)acrylate, N,N,N-trimethylaminododecyl (meth)acrylate, N-[3-(trimethylamino)propyl]methacrylamide and N-[3-(trimethylamino)propyl]acrylamide, N-[3-(dimethylamino)butyl]methacrylamide, N-[8-(trimethylamino)octyl]methacrylamide, N-[12-(trimethylamino)dodecyl]methacrylamide, N-[3-(triethylamino)propyl]methacrylamide and N-[3-(triethylamino)propyl]acrylamide, (meth)acryloyloxyhydroxypropyltrimethylamine, (meth)acryloyloxyhydroxypropyltriethylamine, 3-methyl-1-vinylimidazole and N,N-dimethyl-N,N-diallylamine.

**5.** The use according to any of claims 1 to 4, where the polymers are obtainable starting from mixtures of 3-methyl-1-vinylimidazolium methylsulfate and N,N-dimethyl-N,N-diallylammonium chloride as component (a1).

**6.** The use according to any of claims 1 to 5, where the monomer (a2) is selected from free-radically polymerizable unsaturated primary, secondary and tertiary amines, unsaturated acids and unsaturated halides.

**7.** The use according to any of claims 1 to 6, where the monomer (a2) is an amine selected from the group of compounds consisting of

a) aminoalkyl acrylates, aminoalkyl methacrylates, aminoalkylacrylamides and aminoalkylmethacrylamides of the formula (VIII)

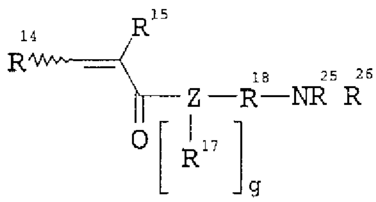

(VIII)

where for $R^{14}$ to $R^{18}$ the definitions given for formula (IV) in claim 3 b) apply, and $R^{25}$ and $R^{26}$ are in each case and independently of one another selected from the group consisting of hydrogen, $C_1$-$C_{40}$ linear- or branched-chain alkyl, formyl, $C_1$-$C_{10}$ linear- or branched-chain acyl, N,N-dimethylaminoethyl, 2-hydroxyethyl, 2-methoxyethyl, 2-ethoxyethyl, hydroxypropyl, methoxypropyl, ethoxypropyl or benzyl,
b) N-vinylimidazoles of the formula IX, where for $R^{19}$ to $R^{21}$, independently of one another, the definitions given for formula (V) in claim 3 c) apply,

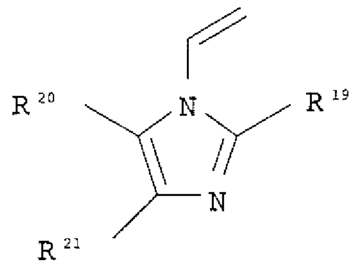

(IX)

c) diallylamines of the formula (X)

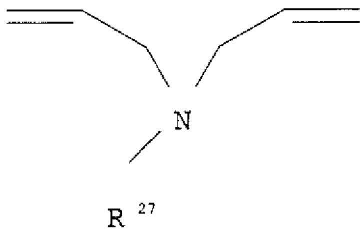

(X)

where $R^{27}$ = hydrogen or $C_1$- to $C_{24}$-alkyl,

d) 1,3-divinylimidazolid-2-one or N-disubstituted vinylamines of the formula (XI):

$$(R^{28})_2N\text{-}(CH_2)_n\text{-}CR^{15}\text{=}CHR^{14} \qquad \text{(XI)}$$

where $R^{14}$, $R^{15}$ and n have the same meanings as in the formulae (IIIa) and (IIIb), and the radicals $R^{28}$ can be selected from the group consisting of hydrogen, $C_1$-$C_{40}$ linear- or branched-chain alkyl radicals, formyl, $C_1$-$C_{10}$ linear- or branched-chain acyl, N,N-dimethylaminoethyl, 2-hydroxyethyl, 2-methoxyethyl, 2-ethoxyethyl, hydroxypropyl, methoxypropyl, ethoxypropyl or benzyl, where, when n=0, both radicals $R^{28}$ are not hydrogen at the same time.

**8.** The use according to any of claims 1 to 7, where the monomer (a2) is selected from N,N-dimethylaminoethyl methacrylate, N-[3-(dimethylamino)propyl]methacrylamide, N-methylaminoethyl methacrylate, N-[3-(methylamino) propyl]methacrylamide, aminoethyl methacrylate and N-[3-aminopropyl]methacrylamide, N-vinylimidazole, 1-vinyl-2-methylvinylimidazole and N,N-diallylamine.

**9.** The use according to any of claims 6 to 8, where the quaternization takes place with an alkyl halide having 1 to 24 carbon atoms, a dialkyl sulfate having 1 to 24 carbon atoms, an alkylene oxide or an epichlorohydrin.

**10.** The use according to claim 6, where the monomer (a2) is an unsaturated halide selected from haloalkyl acrylates or haloalkyl methacrylates.

**11.** The use according to claim 10, where the quaternization takes place with a trialkylamine.

**12.** The use according to any of claims 1 to 11, where the monomer (c) is selected from the group consisting of N-vinyllactams, N-vinylcarboxamides, hydroxyalkyl acrylates, ethylenically unsaturated amides, vinylimidazoles, unsaturated acids and unsaturated amines.

**13.** The use according to any of claims 1 to 12, where the monomer (c) is selected from the group consisting of N-vinylpyrrolidone, N-vinylpiperidone, N-vinylcaprolactam, N-vinylformamide, N-ethyl-N-vinylacetamide or N-methyl-N-vinylacetamide, 2-hydroxyethyl acrylate, 2-hydroxyethyl methacrylate, 2-hydroxypropyl methacrylate, butanediol monoacrylate, acrylamide, methacrylamide, N-vinylimidazole, acrylic acid, maleic acid, methacrylic acid, 2-acrylamido-2-methylpropanesulfonic acid, dimethylaminoethyl acrylate and dimethylaminoethyl methacrylate.

**14.** The use according to any of claims 1 to 13, where the monomer (d) is selected from the group consisting of $C_1$-$C_{10}$-alkyl

esters of monoethylenically unsaturated $C_3$-$C_6$-carboxylic acids, di-$C_1$-$C_{10}$-alkyl esters of ethylenically unsaturated dicarboxylic acids, hydrocarbons having at least one free-radically polymerizable carbon-carbon double bond, vinyl, vinylidene or allyl halides, vinyl, allyl and methallyl esters of $C_1$-$C_{40}$ linear, $C_3$-$C_{40}$ branched-chain or $C_3$-$C_{40}$ carbocyclic carboxylic acids of aliphatic, saturated and unsaturated nature, vinyl, allyl and methallyl ethers of linear or branched, aliphatic alcohols having 2 to 20 carbon atoms.

**15.** The use according to any of claims 1 to 14, where the monomer (d) is selected from the group consisting of methyl acrylate, ethyl acrylate, propyl acrylate, n-butyl acrylate, isobutyl acrylate, t-butyl acrylate, 2-ethylhexyl acrylate, decyl acrylate, methyl methacrylate, ethyl methacrylate, propyl methacrylate, n-butyl methacrylate, isobutyl methacrylate, t-butyl methacrylate, 2-ethylhexyl methacrylate, decyl methacrylate, methyl ethacrylate, ethyl ethacrylate, n-butyl ethacrylate, isobutyl ethacrylate, t-butyl ethacrylate, 2-ethylhexyl ethacrylate, decyl ethacrylate, stearyl acrylate, stearyl (meth)acrylate, preferably styrene, alpha-methylstyrene, tert-butylstyrene, butadiene, isoprene, cyclohexadiene, ethylene, propylene, 1-butene, 2-butene, isobutylene, vinyltoluene, vinyl chloride, vinylidene chloride, allyl chloride, vinyl acetate, vinyl propionate, vinyl butyrate, vinyl valerate, vinyl hexanoate, vinyl 2-ethylhexanoate, vinyl decanoate, vinyl laurate, vinyl stearate, vinyl methyl ether, vinyl ethyl ether, vinyl dodecyl ether, vinyl hexadecyl ether, vinyl stearyl ether, acrylamidoglycolic acid, fumaric acid and crotonic acid.

**16.** The use according to any of claims 1 to 15, where the polyether-containing compound (b) described by the formula I has an average molecular weight of from 500 to 50 000 (number-average) and the variables, independently of one another, have the following meanings:

$R^1$ is hydrogen, $C_1$-$C_6$-alkyl, $R^6$-C(=O)-, $R^6$-NH-C(=O)-;
$R^5$ is hydrogen, $C_1$-$C_6$-alkyl, $R^6$-C(=O)-, $R^6$-NH-C(=O)-;
$R^2$ to $R^4$ are $-(CH_2)_2-$, $-(CH_2)_3-$, $-(CH_2)_4-$, $-CH_2-CH(R^6)-$, $-CH_2-CHOR^7-CH_2-$;
$R^6$ is $C_1$-$C_6$-alkyl;
$R^7$ is hydrogen, $C_1$-$C_6$-alkyl, $R^6$-C(=O)-, $R^6$-NH-C(=O)-;
n is 1;
s is 0;
u is 5 to 500;
v is 0 to 500;
w is 0 to 500.

**17.** The use according to any of claims 1 to 16, where the polyether-containing compound is a polymer, copolymer or block copolymer of at least one compound selected from ethylene oxide and propylene oxide.

**18.** The use of polymers according to any of claims 1 to 17, where the quantitative ratios are as follows:

a1) 3 - 30% by weight
b) 70 - 97% by weight
c) 0 - 15% by weight
d) 0 - 15% by weight

and the percentages by weight of the individual components a1, b and if appropriate c and d add up in each case to 100% by weight.

**19.** The use of the polymers according to any of claims 1 to 18, where the quantitative ratios are as follows:

a1) 4 - 12% by weight
b) 88 - 96% by weight
c) 0% by weight
d) 0% by weight

and the percentages by weight of the individual components a1 and b add up in each case to 100% by weight.

**20.** A cationic polymer obtainable by polymerization of
from 3 to 30% by weight of at least one cationic, quaternary, free-radically polymerizable monomer (a1)
in the presence of from 70 to 97% by weight of at least one polyether-containing compound (b) selected from the group consisting of

compounds of the formula I

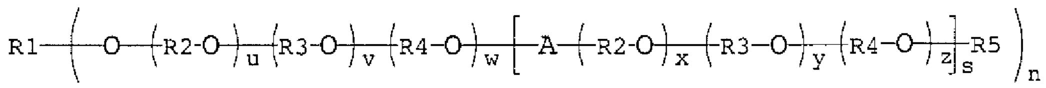

(I)

in which the variables, independently of one another, have the following meanings:

$R^1$ is hydrogen, $C_1$-$C_{24}$-alkyl, $R^6$-C(=O)-, $R^6$-NH-C(=O)-, polyalcohol radical;
$R^5$ is hydrogen, $C_1$-$C_{24}$-alkyl, $R^6$-C(=O)-, $R^6$-NH-C(=O)-;
$R^2$ to $R^4$ are $-(CH_2)_2-$, $-(CH_2)_3-$, $-(CH_2)_4-$, $-CH_2-CH(R^6)-$, $-CH_2-CHOR^7-CH_2-$;
$R^6$ is $C_1$-$C_{24}$-alkyl;
$R^7$ is hydrogen, $C_1$-$C_{24}$-alkyl, $R^6$-C(=O)-, $R^6$-NH-C(=O)-;
A is -C(=O)-O, -C(=O)-B-C(=O)-O, $-CH_2-CH(-OH)-B-CH(-OH)-CH_2-O$, -C (=O)-NH-H-B-NH-C(=O)-O;

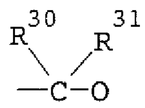 ;

B is $-(CH_2)_t-$, arylene, optionally substituted;
$R^{30}$, $R^{31}$ are hydrogen, $C_1$-$C_{24}$-alkyl, $C_1$-$C_{24}$-hydroxyalkyl, benzyl or phenyl;
n is 1 when $R^1$ is not a polyalcohol radical or
n is 1 to 1 000 when $R^1$ is a polyalcohol radical
s is 0 to 1 000;
t is 1 to 12;
u is 1 to 5 000;
v is 0 to 5 000;
w is 0 to 5 000;
x is 0 to 5 000;
y is 0 to 5 000;
z is 0 to 5 000,

polyether-containing silicone derivatives and
reaction products of polyethyleneimines with alkyene oxides,
if appropriate from 0 to 15% by weight of one or more further free-radically polymerizable monomers (c) with a solubility in water above 60 g/l at 25°C and
if appropriate from 0 to 15% by weight of one or more further free-radically polymerizable monomers (d) with a solubility in water below 60 g/l at 25°C,
where the molar ratio of the sum of the monomers (a1) and (c) to the sum of the monomers (d) is at least 2 to 1,
where the water content in the reaction mixture during the polymerization is less than 20% by weight, and
where the percentages by weight of the individual components a1, b and if appropriate c and d add up in each case to 100% by weight.

**21.** The cationic polymer according to claim 20, where

i) the monomers (a1) are defined as in any of claims 2 to 5 ii) the polyether-containing compound (b) is defined as in any of claims 1, 16, 17,
iii)the monomers (c) are defined as in either claim 12 or 13, and/or
iv) the monomers (d) are defined as in either claim 14 or 15.

**22.** The cationic polymer according to either claim 20 or 21, where the composition of the polymer is defined as in either of claims 18 and 19.

**23.** A process for the preparation of cationic polymers according to any of claims 20 to 22, which comprises polymerizing from 3 to 30% by weight of at least one cationic, quaternary free-radically polymerizable monomer (a1)
in the presence of from 70 to 97% by weight of at least one polyether-containing compound (b) and
if appropriate from 0 to 15% by weight of one or more further free-radically polymerizable monomers (c) with a solubility in water of more than 60 g/l at 25°C and
if appropriate from 0 to 15% by weight of one or more further free-radically polymerizable monomers (d) with a solubility in water of less than 60 g/l at 25°C,
where the molar ratio of the sum of the monomers (a1) and (c) to the sum of the monomers (d) is at least 2 to 1,
where the water content in the reaction mixture during the polymerization is less than 20% by weight, and
where the percentages by weight of the individual components a1, b and if appropriate c and d add up in each case to 100% by weight.

**24.** A hair cosmetic formulation with the following composition:

a) 0.05 - 20% by weight of a cationic polymer corresponding to any of claims 1 to 22
b) 20 - 99.95% by weight of water and/or alcohol
c) 0 - 79.05% by weight of further constituents.

**25.** A hair cosmetic formulation with the following composition:

a) 0.1 - 10% by weight of a cationic polymer according to any of claims 1 to 22
b) 20 - 99.9% by weight of water and/or alcohol
c) 0 - 70% by weight of a propellant
d) 0 - 20% by weight of further constituents.

**26.** A hair cosmetic formulation with the following composition:

a) 0.1 - 10% by weight of a cationic polymer according to any of claims 1 to 22
b) 55 - 94.8% by weight of water and/or alcohol
c) 5 - 20% by weight of a propellant
d) 0.1 - 5% by weight of an emulsifier
e) 0 - 10% by weight of further constituents.

**27.** A hair cosmetic formulation of the following composition:

a) 0.1 - 10% by weight of a cationic polymer according to any of claims 1 to 22
b) 60 - 99.85% by weight of water and/or alcohol
c) 0.05 - 10% by weight of a gel former
d) 0 - 20% by weight of further constituents.

**28.** A hair cosmetic formulation with the following composition:

a) 0.05 - 10% by weight of a cationic polymer according to any of claims 1 to 22,
b) 25 - 94.95% by weight of water
c) 5 - 50% by weight of surfactants
d) 0 - 5% by weight of a further conditioning agent
e) 0 - 10% by weight of further cosmetic constituents.

## Revendications

**1.** Utilisation de polymères cationiques, pouvant être obtenus par polymérisation de
3 à 30 % en poids d'au moins un monomère (a1) polymérisable par voie radicalaire, contenant de l'azote quaternaire, et/ou d'un précurseur direct (a2) de ce même monomère
en présence de 70 à 97 % en poids d'au moins un composé (b) contenant du polyéther, sélectionné dans le groupe constitué de
composés de la formule générale I

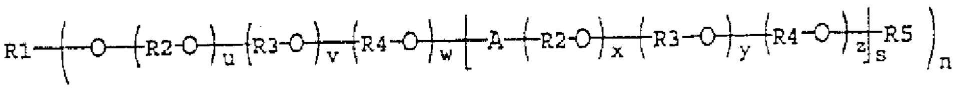

(I)

dans laquelle les variables ont indépendamment les unes des autres la signification suivante :

$R^1$ hydrogène, alkyle en $C_1$-$C_{24}$, $R^6$-C(=O)-, $R^6$-NH-C(=O)-, radical polyalcool ;
$R^5$ hydrogène, alkyle en $C_1$-$C_{24}$, $R^6$-C(=O)-, $R^6$-NH-C(=O)-; $R^2$ à $R^4$ - $(CH_2)_2$-, $(CH_2)_3$-, -$(CH_2)_4$-,-$CH_2$-CH($R^6$)-, -$CH_2$-CHO$R^7$-$CH_2$- ;
$R^6$ alkyle en $C_1$-$C_{24}$ ;
$R^7$ hydrogène, alkyle en $C_1$-$C_{24}$, $R^6$-C(=O)- , -$R^6$-NH- C(=O) - ;
A -C (=O) -O, -C (=O) -B-C (=O) -O, -$CH_2$-CH(-OH)-B-CH(-OH)-$CH_2$-O, -C(=O)-NH-B-NH-C(=O)-O ;

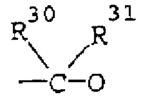 ;

B -($CH_2$)-, aryles, éventuellement substitués ;
$R^{30}$, $R^{31}$ hydrogène, alkyle en $C_1$-$C_{24}$, hydroxyalkyle en $C_1$-$C_{24}$, benzyle ou phényle ;
n 1 si $R^1$ n'est pas un radical polyalcool ou
n 1 à 1000 si $R^1$ est un radical polyalcool
s 0 à 1000 ;
t 1 à 12 ;
u 1 à 5000 ;
v 0 à 5000 ;
w 0 à 5000 ;
x 0 à 5000 ;
y 0 à 5000 ;
z 0 à 5000,

produits dérivés de silicone contenant du polyéther et
produits de réaction de polyéthylènimines avec des oxydes d'alkylène,
éventuellement 0 à 15 % en poids d'un ou plusieurs autres monomères (c) polymérisables par voie radicalaire et ayant une solubilité dans l'eau supérieure à 60 g/l à 25°C et
éventuellement 0 à 15 % en poids d'un ou plusieurs autres monomères (d) polymérisables par voie radicalaire et ayant une solubilité dans l'eau inférieure à 60 g/l à 25°C
dans laquelle la teneur en eau dans le mélange réactionnel pendant la polymérisation est inférieure à 20 % en poids, et dans laquelle, lors d'une utilisation d'un précurseur (a2), celui-ci est converti à la suite de ou pendant la polymérisation au moins en partie en un composé avec de l'azote quaternaire (a2'), et
dans laquelle le rapport molaire de la somme des monomères (a1), (a2') et (c) sur la somme des monomères (d) est d'au moins 2:1, et
dans laquelle les % en poids des composants individuels a1 et/ou a2, b et éventuellement c et d s'additionnent jusqu'à respectivement 100 % en poids,
dans des préparations cosmétiques.

**2.** Utilisation selon la revendication 1, dans laquelle le monomère (a1) et/ou le composé (a2') sont sélectionnés dans le groupe constitué des vinylamines quaternaires, des acrylates de N,N,N-trialkylaminoalkyle et des méthacrylates de N,N,N-trialkylaminoalkyle, des acrylamides de N,N,N-trialkylaminoalkyle et des méthacrylamides de N,N,N-trialkylaminoalkyle, des 3-alkyl-1-vinylimidazoles, des 3-aryl-1-vinylimidazoles, des vinylpyridines quaternaires et des diallylamines quaternaires, ainsi que leurs sels.

**3.** Utilisation selon la revendication 1 ou 2, dans laquelle le monomère (a1) et/ou le composé (a2') sont sélectionnés dans le groupe constitué des

a) vinylamines quaternaires des formules générales (IIIa) ou (IIIb)

$$W\text{-}(CH_2)_n\text{-}CR^{15}\text{=}CHR^{14} \qquad \text{(IIIa)}$$

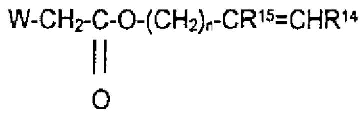

(IIIb)

dans lesquelles

$R^{14}$ et $R^{15}$ sont sélectionnés indépendamment l'un de l'autre dans le groupe constitué d'hydrogène, d'alkyle en $C_1$-$C_8$ linéaire ou ramifié, de méthoxy, d'éthoxy, de 2-hydroxyéthoxy, de 2-méthoxyéthoxy et de 2-éthoxyéthyle, et

n vaut 0, 1 ou 2, et

W est $-N^+(R^{16})_3/X^-$ ou

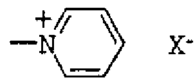

dans laquelle les radicaux $R^{16}$ peuvent être sélectionnés identiques ou différents dans le groupe constitué des radicaux alkyle en $C_1$-$C_{40}$ linéaires ou ramifiés, du formyle, de l'acyle en $C_1$-$C_{10}$ linéaire ou ramifié, du N,N-diméthylaminoéthyle, du 2-hydroxyéthyle, du 2-méthoxyéthyle, du 2-éthoxyéthyle, de l'hydroxypropyle, du méthoxypropyle, de l'éthoxypropyle ou du benzyle, et dans laquelle $X^-$ est un anion,

b) acrylates de N,N,N-trialkylaminoalkyle ou méthacrylates de N,N,N-trialkylaminoalkyle, acrylamides de N,N,N-trialkylaminoalkyle ou méthacrylamides de N,N,N-trialkylaminoalkyle, de la formule générale (IV)

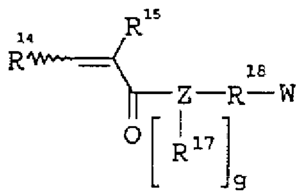

(IV)

dans laquelle $R^{14}$, $R^{15}$ et W ont la même signification que dans les formules générales IIIa et IIIb dans la revendication 3 a), et

$R^{17}$ = hydrogène ou méthyle,

$R^{18}$ = alkylène ou hydroxyalkylène ayant 1 à 24 atomes de C,

Z = azote pour g = 1 ou oxygène pour g = 0,

c) N-vinylimidazoles quaternaires de la formule générale (V)

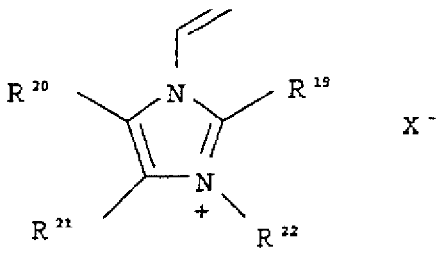

(V)

dans laquelle
$R^{19}$ à $R^{21}$ représentent indépendamment les uns des autres de l'hydrogène, de l'alkyle en $C_1$-$C_4$, de l'hydroxyalkyle en $C_1$-$C_4$ ou du phényle ; et
$R^{22}$ représente de l'alkyle en $C_1$-$C_4$, de l'hydroxyalkyle en $C_1$-$C_4$ ou du phényle ; et
et $X^-$ représente un anion,
d) vinylpyridines quaternaires de la formule générale (VI)

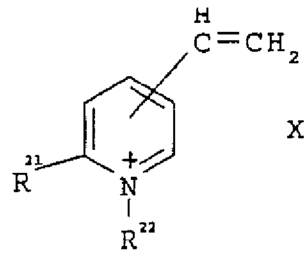

X⁻ (VI)

dans laquelle $R^{21}$ , $R^{22}$ et $X^-$ ont la même signification que dans la formule générale (V) dans la revendication 3 c),
e) diallylamines quaternaires de la formule générale (VII)

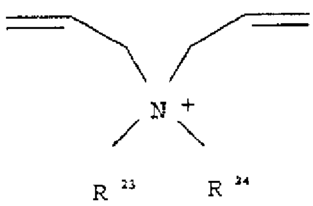

X⁻ (VII)

dans laquelle $R^{23}$ et $R^{24}$ peuvent être respectivement et indépendamment l'un de l'autre de l'alkyle en $C_1$-$C_{24}$ et $X^-$ représente un anion.

**4.** Utilisation selon l'une quelconque des revendications 1 à 3, dans laquelle le monomère (a1) et/ou le composé (a2') sont sélectionnés parmi le (méth)acrylate de N,N,N-triméthylaminométhyle, le (méth)acrylate de N,N,N-triéthylaminométhyle, le (méth) acrylate de N, N, N-triméthylaminoéthyle, le (méth)acrylate de N,N,N-triéthylaminoéthyle, le (méth)acrylate de N,N,N-triméthylaminobutyle, le (méth) acrylate de N,N,N-triéthylaminobutyle, le (méth) acrylate de N,N,N-triméthylaminohexyle, le (méth) acrylate de N, N, N-triméthylaminooctyle, le (méth)acrylate de N,N,N-triméthylaminododécyle, le méthacrylamide de N-[3-(triméthylamino)propyle] et l'acrylamide de N-[3-(triméthylamino)propyle], le méthacrylamide de N-[3-(diméthylamino)butyle], le méthacrylamide de N-[8-(triméthylamino)octyle], le méthacrylamide de N-[12-(triméthylamino)dodécyle], le méthacrylamide de N-[3-(triéthylamino)propyle] et l'acrylamide de N-[3-(triéthylamino)propyle], la triméthylamine de (méth)acryloyloxyhydroxypropyle, la triéthylamine de (méth)acryloyloxyhydroxypropyle, le 3-méthyl-1-vinylimidazole et la N,N-diméthyl-N,N-diallylamine.

**5.** Utilisation selon l'une quelconque des revendications 1 à 4, dans laquelle les polymères peuvent être obtenus à partir de mélanges de méthylsulfate de 3-méthyl-1-vinylimidazolium et de chlorure de N,N-diméthyl-N,N-diallylammonium en tant que composant (a1).

**6.** Utilisation selon l'une quelconque des revendications 1 à 5, dans laquelle le monomère (a2) est sélectionné parmi des amines primaires, secondaires et tertiaires insaturées polymérisables par voie radicalaire, des acides insaturés et des halogénures insaturés.

**7.** Utilisation selon l'une quelconque des revendications 1 à 6, dans laquelle le monomère (a2) est une amine sélectionnée dans le groupe de composés constitué des

a) acrylates d'aminoalkyle, méthacrylates d'aminoalkyle, acrylamides d'aminoalkyle et méthacrylamides d'aminoalkyle de la formule générale (VIII)

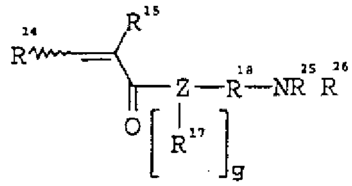

(VIII)

dans laquelle les définitions données pour la formule (IV) dans la revendication 3 b) valent pour $R^{14}$ à $R^{18}$ et dans laquelle $R^{25}$ ou $R^{26}$ sont sélectionnés respectivement et indépendamment l'un de l'autre dans le groupe constitué de l'hydrogène, de l'alkyle en $C_1$-$C_{40}$ linéaire ou ramifié, du formyle, de l'acyle en $C_1$-$C_{10}$ linéaire ou ramifié, du N,N-diméthylaminoéthyle, du 2-hydroxyéthyle, du 2-méthoxyéthyle, du 2-éthoxyéthyle, de l'hydroxypropyle, du méthoxypropyle, de l'éthoxypropyle ou du benzyle,

b) N-vinylimidazoles de la formule générale IX, dans laquelle les définitions données pour la formule (V) dans la revendication 3 c) valent pour $R^{19}$ à $R^{21}$ indépendamment l'un de l'autre,

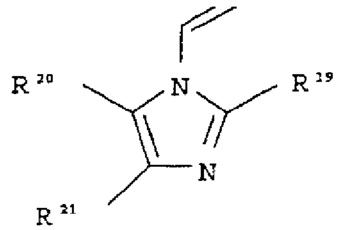

(IX)

c) diallylamines de la formule générale (X)

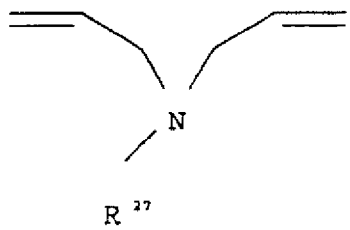

(X)

avec $R^{27}$ = hydrogène ou alkyle en $C_1$-$C_{24}$,

d) 1,3-divinylimidazolid-2-one ou vinylamines disubstituées en N de la formule générale (XI) :

$$(R^{28})_2N\text{-}(CH_2)_n\text{-}CR^{15}\text{=}CHR^{14} \qquad \textbf{(XI)}$$

dans laquelle $R^{14}$, $R^{15}$ et n ont la même signification que dans les formules (IIIa) et (IIIb), et les radicaux $R^{28}$ peuvent être sélectionnés dans le groupe constitué de l'hydrogène, des radicaux alkyle en $C_1$-$C_{40}$ linéaires ou ramifiés, du formyle, de l'acyle en $C_1$-$C_{10}$ linéaire ou ramifié, du N,N-diméthylaminoéthyle, du 2-hydroxyéthyle, du 2-méthoxyéthyle, du 2-éthoxyéthyle, de l'hydroxypropyle, du méthoxypropyle, de l'éthoxypropyle ou du benzyle, dans laquelle on admet que, quand n = 0, les deux radicaux $R^{28}$ ne sont pas simultanément de l'hydrogène.

**8.** Utilisation selon l'une quelconque des revendications 1 à 7, dans laquelle le monomère (a2) est sélectionné parmi le méthacrylate de N,N-diméthylaminoéthyle, le méthacrylamide de N-[3-(diméthylamino)propyle], le méthacrylate de N-méthylaminoéthyle, le méthacrylamide de N-[3-(méthylamino)propyle], le méthacrylate d'aminoéthyle et le méthacrylamide de N-[3-aminopropyle], le N-vinylimidazole, le vinylimidazole de 1-vinyl-2-méthyle et la N,N-diallylamine.

**9.** Utilisation selon l'une quelconque des revendications 6 à 8, dans laquelle la quaternisation a lieu avec un halogénure d'alkyle ayant 1 à 24 atomes de C, un sulfate de dialkyle ayant 1 à 24 atomes de C, un oxyde d'alkylène ou une épichlorhydrine.

**10.** Utilisation selon la revendication 6, dans laquelle le monomère (a2) est un halogénure insaturé choisi parmi les acrylates d'halogénoalkyle ou les méthacrylates d'halogénoalkyle.

**11.** Utilisation selon la revendication 10, dans laquelle la quaternisation a lieu avec une trialkylamine.

**12.** Utilisation selon l'une quelconque des revendications 1 à 11, dans laquelle le monomère (c) est sélectionné dans le groupe constitué des N-vinyllactames, des amides d'acide N-vinylcarboxylique, des acrylates d'hydroxyalkyle, des amides éthyléniquement insaturées, des vinylimidazoles, des acides insaturés et des amines insaturées.

**13.** Utilisation selon l'une quelconque des revendications 1 à 12, dans laquelle le monomère (c) est sélectionné dans le groupe constitué de la N-vinylpyrrolidone, de la N-vinylpipéridone, du N-vinylcaprolactame, du N-vinylformamide, du N-éthyl-N-vinylacétamide ou du N-méthyl-N-vinylacétamide, de l'acrylate de 2-hydroxyéthyle, du méthacrylate de 2-hydroxyéthyle, du méthacrylate de 2-hydroxypropyle, du monoacrylate de butanediol, de l'acrylamide, du méthacrylamide, du N-vinylimidazole, de l'acide acrylique, de l'acide maléique, de l'acide méthacrylique, de l'acide 2-acrylamido-2-méthylpropanesulfonique, de l'acrylate de diméthylaminoéthyle et du méthacrylate de diméthylamino.

**14.** Utilisation selon l'une quelconque des revendications 1 à 13, dans laquelle le monomère (d) est sélectionné dans le groupe constitué d'esters alkyliques en $C_1$-$C_{10}$ d'acides carboxyliques en $C_3$-$C_6$ monoéthyléniquement insaturés, d'esters dialkyliques en $C_1$-$C_{10}$ d'acides dicarboxyliques éthyléniquement insaturés, d'hydrocarbures ayant au moins une liaison double carbone-carbone polymérisable par voie radicalaire, d'halogénures de vinyle, de vinylidène ou d'allyle, d'esters vinyliques, allyliques ou méthallyliques d'acides carboxyliques en $C_1$-$C_{40}$ linéaires, en $C_3$-$C_{40}$ ramifiés ou en $C_3$-$C_{40}$ carbocycliques, de nature aliphatique, saturée et insaturée, d'éthers vinyliques, allyliques et méthallyliques d'alcools aliphatiques, linéaires ou ramifiés, ayant 2 à 20 atomes de C.

**15.** Utilisation selon l'une quelconque des revendications 1 à 14, dans laquelle le monomère (d) est sélectionné dans le groupe constitué de l'acrylate de méthyle, de l'acrylate d'éthyle, de l'acrylate de propyle, de l'acrylate de n-butyle, de l'acrylate d'isobutyle, de l'acrylate de t-butyle, de l'acrylate de 2-éthylhexyle, de l'acrylate de décyle, du méthacrylate de méthyle, du méthacrylate d'éthyle, du méthacrylate de propyle, du méthacrylate de n-butyle, du méthacrylate d'isobutyle, du méthacrylate de t-butyle, du méthacrylate de 2-éthylhexyle, du méthacrylate de décyle, de l'éthacrylate de méthyle, de l'éthacrylate d'éthyle, de l'éthacrylate de n-butyle, de l'éthacrylate d'isobutyle, de l'éthacrylate de t-butyle, de l'éthacrylate de 2-éthylhexyle, de l'éthacrylate de décyle, de l'acrylate de stéaryle, du (méth) acrylate de stéaryle, de manière préférée du styrène, de l'alpha-méthylstyrène, du tert-butylstyrène, du butadiène, de l'isoprène, du cyclohexadiène, de l'éthylène, du propylène, du 1-butène, du 2-butène, de l'isobutylène, du vinyltoluène, du chlorure de vinyle, du chlorure de vinylidène, du chlorure d'allyle, de l'acétate de vinyle, du propionate de vinyle, du butyrate de vinyle, du valérate de vinyle, de l'hexanoate de vinyle, de l'hexanoate de vinyl-2-éthyle, du décanoate de vinyle, du laurate de vinyle, du stéarate de vinyle, du méthyléther de vinyle, de l'éthyléther de vinyle, du dodécyléther de vinyle, de l'hexadécyléther de vinyle, du stéaryléther de vinyle, de l'acide acrylamidoglycolique, de l'acide fumarique et de l'acide crotonique.

**16.** Utilisation selon l'une quelconque des revendications 1 à 15, dans laquelle le composé (b) contenant du polyéther et décrit par la formule générale I a un poids moléculaire moyen de 500 à 50 000 (selon la moyenne numérique) et les variables ont indépendamment les unes des autres la signification suivante :

$R^1$ hydrogène, alkyle en $C_1$-$C_6$, $R^6$-C(=O)-, $R^6$-NH-C(=O) - ;
$R^5$ hydrogène, alkyle en $C_1$-$C_6$, $R^6$-C(=O)-, $R^6$-NH-C (=O)- ;
$R^2$ à $R^4$ $-(CH_2)_2-$, $-(CH_2)_3-$, $-(CH_2)_4-$, $-CH_2-CH(R^6)-$, $-CH_2-CHOR^7-CH_2-$ ,
$R^6$ alkyle en $C_1$-$C_6$ ;
$R^7$ hydrogène, alkyle en $C_1$-$C_6$, $R^6$-C(=O)-, $R^6$-NH-C(=O)-;
n 1 ;
S 0;
u 5 à 500 ;
v 0 à 500 ;
w 0 à 500.

**17.** Utilisation selon l'une quelconque des revendications 1 à 16, dans laquelle le composé contenant du polyéther est un polymère, un copolymère ou un copolymère en bloc d'au moins un composé sélectionné parmi l'oxyde d'éthylène et l'oxyde de propylène.

**18.** Utilisation de polymères selon l'une quelconque des revendications 1 à 17, dans laquelle les proportions sont

a1) 3 à 30 % en poids
b) 70 à 97 % en poids
c) 0 à 15 % en poids
d) 0 à 15 % en poids

et les % en poids des composants individuels a1, b et éventuellement c et d s'additionnent jusqu'à respectivement 100 % en poids.

**19.** Utilisation de polymères selon l'une quelconque des revendications 1 à 18, dans laquelle les proportions sont

a1) 4 à 12 % en poids
b) 88 à 96 % en poids
c) 0 % en poids
d) 0 % en poids

et les % en poids des composants individuels a1 et b s'additionnent jusqu'à 100 % en poids.

**20.** Polymère cationique pouvant être obtenu par la polymérisation de
3 à 30 % en poids d'au moins un monomère (a1) cationique, quaternaire, polymérisable par voie radicalaire
en présence de 70 à 97 % en poids d'au moins un composé (b) contenant du polyéther sélectionné dans le groupe constitué de
composés de la formule générale I

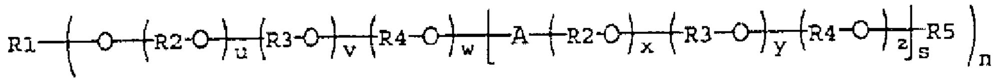

(I)

dans laquelle les variables ont indépendamment les unes des autres la signification suivante :

$R^1$ hydrogène, alkyle en $C_1$-$C_{24}$, $R^6$-C(=O)-, $R^6$-NH-C(=O)-, radical polyalcool ;
$R^5$ hydrogène, alkyle en $C_1$-$C_{24}$, $R^6$-C(=O)-, $R^6$-NH-C(=O)-;
$R^2$ à $R^4$ $-(CH_2)_2-$, $-(CH_2)_3-$, $-(CH_2)_4-$, $-CH_2-CH(R^6)-$, $-CH_2CHOR^7-CH_2-$;
$R^6$ alkyle en $C_1$-$C_{24}$;
$R^7$ hydrogène, alkyle en $C_1$-$C_{24}$, $R^6$-C(=O)-, -$R^6$-NH-C (=O) - ;
A -C(=O)-O, -C(=O)-B-C(=O)-O, $-CH_2-CH(-OH)-B-CH(-OH)-CH_2-O$, -C(=O)-NH-B-NH-C(=O)-O;

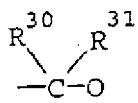
;

B $-(CH_2)-$, aryles, éventuellement substitués ;
$R^{30}$, $R^{31}$ hydrogène, alkyle en $C_1$-$C_{24}$ hydroxyalkyle en $C_1$-$C_{24}$, benzyle ou phényle ;
n 1 si $R^1$ n'est pas un radical polyalcool ou
n 1 à 1000 si $R^1$ est un radical polyalcool
s 0 à 1000 ;
t 1 à 12 ;
u 1 à 5000 ;

v 0 à 5000 ;
w 0 à 5000 ;
x 0 à 5000 ;
y 0 à 5000 ;
z 0 à 5000,

produits dérivés de silicone contenant du polyéther et
produits de réaction de polyéthylènimines avec des oxydes d'alkylène,
éventuellement 0 à 15 % en poids d'un ou plusieurs autres monomères (c) polymérisables par voie radicalaire et ayant une solubilité dans l'eau supérieure à 60 g/l à 25°C et
éventuellement 0 à 15 % en poids d'un ou plusieurs autres monomères (d) polymérisables par voie radicalaire et ayant une solubilité dans l'eau inférieure à 60 g/l à 25°C,
dans lequel le rapport molaire de la somme des monomères (a1) et (c) sur la somme des monomères (d) est d'au moins 2:1,
dans lequel la teneur en eau dans le mélange réactionnel pendant la polymérisation est inférieure à 20 % en poids, et dans lequel les % en poids des composants individuels a1, b et éventuellement c et d se complètent jusqu'à respectivement 100 % en poids.

**21.** Polymère cationique selon la revendication 20, dans lequel

i) les monomères (a1) sont définis selon l'une des revendications 2 à 5
ii) le composé (b) contenant du polyéther est défini selon l'une des revendications 1, 16, 17,
iii) les monomères (c) sont définis selon l'une des revendications 12 ou 13, et/ou
iv) les monomères (d) sont définis selon l'une des revendications 14 ou 15.

**22.** Polymère cationique selon l'une quelconque des revendications 20 ou 21, dans lequel la composition du polymère est définie selon l'une des revendications 18 ou 19.

**23.** Procédé de préparation de polymères cationiques, selon l'une quelconque des revendications 20 à 22, **caractérisé en ce que**
3 à 30 % en poids d'au moins un monomère (a1) cationique, quaternaire, polymérisable par voie radicalaire, sont polymérisés
en présence de 70 à 97 % en poids d'au moins un composé (b) contenant du polyéther et
éventuellement 0 à 15 % en poids d'un ou plusieurs autres monomères (c) polymérisables par voie radicalaire et ayant une solubilité dans l'eau supérieure à 60 g/l à 25°C et
éventuellement 0 à 15 % en poids d'un ou plusieurs autres monomères (d) polymérisables par voie radicalaire et ayant une solubilité dans l'eau inférieure à 60 g/l à 25°C,
dans lequel le rapport molaire de la somme des monomères (a1) et (c) sur la somme des monomères (d) est d'au moins 2:1,
dans lequel la teneur en eau dans le mélange réactionnel pendant la polymérisation est inférieure à 20 % en poids, et dans lequel les % en poids des composants individuels a1, b et éventuellement c et d se complètent jusqu'à respectivement 100 % en poids.

**24.** Formulation cosmétique capillaire, composée comme suit :

| | | |
|---|---|---|
| a) | 0,05 à 20 % en poids | d'un polymère cationique selon l'une quelconque des revendications 1 à 22 |
| b) | 20 à 99,95 % en poids | d'eau et/ou d'alcool |
| c) | 0 à 79,05 % en poids | d'autres ingrédients. |

**25.** Formulation cosmétique capillaire, composée comme suit :

| | | |
|---|---|---|
| a) | 0,1 à 10 % en poids | d'un polymère cationique selon l'une quelconque des revendications 1 à 22 |
| b) | 20 à 99,9 % en poids | d'eau et/ou d'alcool |
| c) | 0 à 70 % en poids | d'un agent gonflant |

(suite)

| | | |
|---|---|---|
| d) | 0 à 20 % en poids | d'autres ingrédients. |

**26.** Formulation cosmétique capillaire, composée comme suit:

| | | |
|---|---|---|
| a) | 0,1 à 10 % en poids | d'un polymère cationique selon l'une quelconque des revendications 1 à 22 |
| b) | 55 à 94,8 % en poids | d'eau et/ou d'alcool |
| c) | 5 à 20 % en poids | d'un agent gonflant |
| d) | 0,1 à 5 % en poids | d'un émulsifiant |
| e) | 0 à 10 % en poids | d'autres ingrédients. |

**27.** Formulation cosmétique capillaire, composée comme suit :

| | | |
|---|---|---|
| a) | 0,1 à 10 % en poids | d'un polymère cationique selon l'une quelconque des revendications 1 à 22 |
| b) | 60 à 99,85 % en poids | d'eau et/ou d'alcool |
| c) | 0,05 à 10 % en poids | d'un gélifiant |
| d) | 0 à 20 % en poids | d'autres ingrédients. |

**28.** Formulation cosmétique capillaire, composée comme suit :

| | | |
|---|---|---|
| a) | 0,05 à 10 % en poids | d'un polymère cationique selon l'une quelconque des revendications 1 à 22 |
| b) | 25 à 94,95 % en poids | d'eau |
| c) | 5 à 50 % en poids | de tensioactifs |
| d) | 0 à 5 % en poids | d'un autre agent de conditionnement |
| e) | 0 à 10 % en poids | d'autres ingrédients cosmétiques. |